# EUROPEAN PATENT APPLICATION

(11) **EP 1 890 155 A1**
(43) Date of publication of application: **20.02.2008**
(21) Application number: 06119102.9
(22) Date of filing: 17.08.2006
(51) Int. Cl.: G01N 33/86, A61B 5/15

(54) **Methods and means for determining platelet function and diagnosing platelet-related and cardiovascular disorders**

(71) Applicant: F. Hoffmann-La Roche AG, 4070 Basel (CH); Roche Diagnostics GmbH, 68305 Mannheim (DE)
(72) Inventor: Spanuth, Eberhard, 69221 Dossenheim (DE); Ivandic, Boris, 80935 München (DE)
(74) Representative: Dick, Alexander

(57) **Abstract**

The invention provides a method for diagnosing platelet (thrombocyte) function comprising the steps of (a) obtaining a blood platelet-containing sample from a subject, and (b) preferably keeping the sample under conditions capable of inhibiting plasmatic coagulation and/or fibrin formation, (c) bringing a marker of platelet function derived from the platelets, or a variant of such marker into solution, wherein at least steps b) and c) are carried out in a blood sampling device and/or wherein additionally the level of the marker of platelet function in the solution is measured. Preferably, the marker of platelet function is brought into solution by activating the platelets in vitro or by means of proteolysis. Preferred markers of platelet function include CD40L, sCD40L, P-selectin, soluble P-selectin, GP-IV, soluble GP-IV, GP-V, soluble GP-V, GP-VI, soluble GP-VI, CD63, platelet factor-4 (PF-4), β-Thromboglobulin (β-TG), or variants thereof. The method provides for diagnosis or determining of platelet hypo- or hyperfunction, cardiovascular disorders and any disorders associated with platelet hypo- or hyperfunction.

## Description

The present invention relates to the diagnosis or determination of dysfunction of blood platelet, particularly platelet hypo- or hyperfunction, and to the diagnosis of platelet-related disorders and cardiovascular disorders. More particularly, the present invention relates to methods and means for sample preparation in the context of such diagnosis.

Under physiological conditions, blood circulates in a closed system of vessels without interruptions of flow and without any blood escaping into surrounding tissues. If the integrity of the wall of a blood vessel is impaired, e.g. by traumatic injury, blood may flow into the surroundig tissue or out of a wound. In a process called hemostasis, the loss of blood is usually restricted by blood platelets (also known as thrombocytes), which - in combination with soluble components of the of plasma - form a clot that closes the site of injury and stops the bleeding.

The mechanisms of hemostasis must be carefully controlled, as any disorder of the coagulation system will result either in excess bleeding in the case of injury (if hemostasis is insufficient) or in the formation of clots in otherwise healthy vessels (if hemostasis is excessively active) in hemostasis. Blood platelets play a key role in initiating and localizing clot formation, and consequently a large number of pathological conditions is associated with dysfunctions of blood platelets. E.g. platelet hypofunction is associated with an increased risk of bleeding, whereas platelet hyperfunction will increase the risk of microthromboembolization, stroke or acute coronary syndrome. Furthermore, in certain pathological conditions, platelets are stimulated and the degree of platelet stimulation may itself be of diagnostic significance, e.g. an indicator of the risk of cardiovascular complications.

Therefore, methods and means for easy and reliable diagnosis of platelet activation and platelet dysfunctions are desirable.

Known methods for determining platelet function include optical aggregometry and impedance aggregometry (see e.g. the special textbook by Gawaz, M.: Blood platelets: physiology, pathophysiology, membrane receptors, antiplatelet priniciples, and therapy for atherothrombotic diseases. - Stuttgart; New York: Thieme, 2001. Pages 44-48).

In optical aggregometry, a sample of platelet-rich citrated plasma or a suspension of washed platelets is inserted into a light transmission photometer and stirred. After the addition of a platelet activator, the level of light transmission is monitored over time. Upon activation, the platelets start to form larger aggregates. This reduces the number of particles in the sample and results in an increase of light transmission. The faster the aggregates form, the higher is the aggregability of the platelets, i.e. the ability to form blood clots.

Impedance aggregometry is also based on the formation of aggregates from activated platelets. Instead of measuring the optical density of the sample, the electrical impedance of the sample is measured. In contrast to optical aggregometry, whole blood can be used for impedance aggregometry.

WO 99/14595 (Accumetrics Inc.) discloses a device for carrying out an aggregometric analysis of blood platelets.

Also known is a platelet function analyzer (e.g. PFA-100, Dade Behring) that measures the time taken for blood, drawn through a fine capillary, to block a membrane coated with collagen and epinephrine (CEPI) or collagen and ADP (CADP). This is referred to as the Closure Time (CT) and is measured in seconds. The test is therefore a combined measure of platelet adhesion and aggregation.

All of these tests require a considerable amount of individual handling, which does not allow for high-throughput analysis. Specially trained personnel and special analyzers are required to carry out the methods, in the case of some methods restricting analysis to well-equipped clinics with special laboratories for blood analysis. The samples used for analyis are not stable and should be analyzed within less than 3 to 4 hours after obtaining the sample. Thus, transport over longer distances is difficult if not impossible.

The measurement of certain biomarkers (including platelet factor 4 (PF-4), β-thromboglobulin (β-TG), P-Selectin, glycoprotein V (GP-V), and glycoprotein IV (GP-IV)) is known as such. For example, sCD40L (soluble CD40 ligand) is a 18 kDa soluble protein found in blood. sCD40L is released by proteolysis from CD40L (CD40 ligand, also known as CD154), a 33 kDa type II transmembrane protein, which belongs to the TNF superfamily of transmembrane proteins.

It has been suggested that 95% of sCD40L detectable in blood originates from blood platelets (Andre, P., et al. (2002): CD40L stabilizes arterial thrombi by a B3 integrin-dependent mechanism. Nature Medicine, vol. 8, 247-52). Preliminary clinical studies indicate that patients with acute coronary syndrome and an increased rate of cardiovascular complications show higher levels of sCD40L in blood than patients with more favourable prognosis.

However, recent investigations have revealed problems of preanalytics in measuring sCD40L.

Ahn et al. have described that the measured level of sCD40L is strongly dependent on the sample material (serum, plasma, or platelet-rich plasma) and on the conditions of sample processing and the temperature and centrifugation conditions (Ahn, E.R., et al. (2004). Differences of soluble CD40L in sera and plasma: Implications on CD40L assay as marker of thrombotic risk. Thromb Res, vol. 114, pp. 143-48). It can be concluded from this study that storage of serum samples on ice or the use of platelet-poor plasma minimize the release of sCD40L ex vivo and better represent sCD40L in vivo.

Mason et al. have described that only surface expression of CD40L compared with platelet-derived (plasma) or total (serum) CD40L proved a reliable marker of platelet function in patients who had stable cardiovascular disease and patients who had unstable cardiovascular disease (Mason, P.J., et al. (2005). Plasma, serum, and platelet expression of CD40 ligand in adults with cardiovascular disease. Am J Cardiol, vol. 96, pp. 1365-69).

Otterdal et al. describe that there seems to be an increase of surface exposure of CD40L after stimulation, but it is still unclear whether there is an increase in surface expression of CD40L after platelet activation. The finding of Otterdal allow no clear conclusion and it is possible that some findings are related to an arifact of aggregation (see e.g. Discussion in Otterdal, K., Pedersen, T.M., Solum, N.O. (2004) Release of soluble CD40 ligand after platelet activation - Studies on the solubilization phase. Thrombosis Resaerch, vol. 114, pp. 167-177).

Weber et al. have come to the conclusion that plasma, but not serum, samples are appropriate for sCD40L measurements and that preanalytic conditions are critical in the assessment of sCD40L concentrations (Weber, M., Rabenau, B., Stanisch, M. et al. (2006). Influence of Sample Type and Storage Conditions on Soluble CD40 Ligand Assessment. Clin Chem, vol. 52(5), pp. 888-91).

In summary, the studies on preanalytics have cast doubt on whether sCD40L is a marker suitable for clinical routine measurement, as measurement would require a level of standardization which is difficult, if not impossible, to achieve in routine clinical practice.

Therefore, there is a need to improve the diagnosis of platelet function. Particularly, there is a need to reduce the amount of individual handling and to enable high-throughput analysis. Preferably, diagnosis should be easy and reliable, and the samples used for analysis should have improved stability.

The object of the invention is solved by a method for diagnosing platelet function comprising the steps of
a) obtaining a blood platelet-containing sample from a subject,
b) preferably keeping the sample under conditions capable of inhibiting coagulation and/or fibrin formation, and
c) bringing a marker of platelet function derived from the platelets into solution, preferably by activating the platelets in the sample,
wherein at least steps b) and c) are carried out in a blood sampling device and/or wherein additionally the level of the marker of platelet function is measured in the solution and/or wherein step c) is carried out immediately after step a).

One of the findings in the course of the invention is that platelet dysfunctions or the level of in vivo platelet stimulation can be diagnosed by activating the platelets in a sample and measuring the level of a marker of platelet function, e.g. sCD40L. Thus, the method allows for in vitro assessment of the degree of in vivo platelet stimulation and can also be used for diagnosing the degree of in vivo platelet stimulation. In a non-limiting embodiment of the invention, any CD40L which has been exposed to the surface of the platelets is solubilized, e.g. by shedding, as sCD40L from the platelet surface into solution upon activation of the platelets in the sample. It has been found that markers which are releasable from the platelets, particularly from the platelet membrane, upon activation in vitro are reliable markers of platelet function and can serve to assess platelet function. Furthermore, the release can be stopped, e.g. by centrifugation of the sample or by contacting the sample with an inhibitor of platelet activation, resulting in a stable sample which can be stored or transported until the level of the marker of platelet function is measured.

In the context of the invention it has been found that previously any measurements e.g. of sCD40L were strongly influenced by methods of obtaining the sample and by subsequent treatment of the sample (e.g. time or temparature of incubation before the marker was measured). It was further found in the context of the invention that if any uncontrolled activation of the platelets in vitro was avoided (e.g. by obtaining the sample in a CTAD cocktail (citrate, theophyllin, adenosine, dipyramidole) and by keeping the sample on ice) there were no significant differences between the levels of sCD40L measured in samples from healthy subjects and patients suffering from acute coronary syndrome. However, it was found unexepectedly that bringing a marker present on the platelet surface into solution is a good indicator of platelet function or platelet stimulation in vivo and is a good indicator of disorders associated with platelet function or platelet stimulation.

If in the respective jurisdiction a method according to the invention is considered to relate also to embodiments which are exempt from patentability or industrial applicability due to relating to a diagnostic method practised on the human or animal body, then such included embodiment shall be exempt from the scope of protection.

The blood platelet sample may be any kind of sample containing blood platelets, e.g. it may be a sample of whole blood (e.g. venous blood or capillary blood). The sample may also be derived from a blood sample which has been further processed, e.g. it may be platelet-rich plasma. Preferably, the platelets in the sample have not been significantly activated or during such further processing. Preferably, there should be no significant coagulation taking place during such further processing. The terms "significantly" and "significant" in this context are understood by the person skilled in the art and mean that any such activation or coagulation should not be of such kind or extent that it renders the sample unsuitable for the diagnosis of interest (as laid out elsewhere in this specification).

The amount of sample may be in any range deemed appropriate and preferably allows reliable measurement of the marker of platelet function. For example, the amount of the sample may be in the range of 1 µl to 500 ml, 10 µl to 100 ml, 100 µl to 50 ml, 0.5 ml to 20 ml, and/or 1 ml to 15 ml. The precise size depends on the purpose or device, e.g. in the case of measurement with point-of-care devices a range of 1 µl to 5 ml, or 2 µl to 2 ml, 2 µl to 500 µl may be considered. Small amounts of sample can be obtained easily by use of capillary blood.

Methods for obtaining blood platelet-containing samples are known to the person skilled in the art. The sample may be obtained by any means deemed appropriate, e.g. by drawing a blood sample from a vein or artery. Also capillary blood may be used. The sample may drawn by means of a needle or a lancet. Preferably, if a venous blood sample is drawn, the first 2 ml of blood should be discarded. Preferably no or only little venous occlusion should be used, in order to avoid stimulation of platelets by venous occlusion. Therefore, preferably the use of a tourniquet should be minimized, preferably to less than a minute.

The method comprises the step of bringing a marker of platelet function derived from the platelets into solution. Preferably, the marker is derived from the platelet surface. This step can be carried out by any means deemed appropriate by the person skilled in the art, e.g. by adding a protease capable of cleaving of a fragment of the marker exposed on the platelet surface or by activating the platelets in the sample.

In a preferred embodiment, the step of bringing the marker of platelet function into solution is carried out immediately after obtaining the blood platelet-containing sample. Therefore, the object of the invention is also solved by a method for diagnosing platelet function comprising the steps of (a) obtaining a blood platelet-containing sample from a subject, and (b) preferably keeping the sample under conditions capable of inhibiting coagulation and/or fibrin formation, and (c) immediately bringing a marker of platelet function derived from the platelets into solution, preferably by activating the platelets in the sample. Immediately bringing the marker of platelet function into solution has the advantage that influences of any uncontrolled activation of the platelets e.g. by different methods of obtaining or treating the sample are further minimized.

The term "immediately" in this context is readily understood by the person skilled in the art. Preferably, "immediately" means that any undue delay should be avoided, in particular that any delay should be avoided which results in such uncontrolled activation of platelets in vitro that diagnosis is not reliable anymore. However, a certain delay may be acceptable if it occurs in a standardized manner in all samples analyzed. The person skilled in the art is able to determine whether a particular delay is acceptable or not, e.g. by analyzing samples obtained from healthy subjects and by determining whether any deviations between different samples are considered to be still acceptable or not. Example 6 shows the influence of delayed processing after keeping the samples at room temperature. It is evident that the time between obtaining the sample and bringing the marker into solution is preferably standardized in order to obtain comparable baseline levels. However, the influence of delayed processing may also be minimized by keeping the sample on ice. More particularly, the term "immediately" relates to starting the step of bringing the marker into solution within equal or less than 30, 10, 5, 4, 3, 2, or 1 minute of after obtaining the sample. Most particularly, the term "immediately" relates to starting the step of bringing the marker into solution within equal or less than 50, 40, 30, 20, 10, or 5 seconds after obtaining the sample. Immediately starting the step of bringing the marker into solution can be conveniently achieved for example if a means or agent capable of bringing the marker of platelet function into solution (e.g. a platelet activator or a protease) is already present in a blood sampling device or blood sampling tube. In this case, said means or agent is able to act immediately on the sample obtained from the subject.

As discussed elsewhere in the specification, the invention takes advantage of certain markers, particularly biochemical markers, e.g. CD40L, p-Selectin, GP-IV, GP-V, CD63 (granulophysin), or GP-VI(p62). These markers are exposed on the platelet surface upon stimulation of the platelets in vivo. Fragments of such surface-exposed markers can be brought into solution, for example by means of proteolysis. The fragments can be measured in solution by any means known to the person skilled in the art. By measuring the level of a thus cleaved-off fragment in solution, the degree of surface exposure of the respective marker on the platelets can be concluded. Preferably, the fragments cleaved off are of a size measurable by a suitable means, e.g. an antibody. Therefore, the size and/or sequence of the fragment should be specific for the respective marker to be determined. Depending on the particular sequence, a fragment is preferably of a size equal or larger than 4, 6, 8, 10, 12, 15, 18, 21, 25, or 30 amino acids. For example, suitable epitopes recognized by antibodies are larger than 10 amino acids. Smaller fragments (such as 4 amino acids or 6 amino acids in length) can be measured e.g. by means of mass spectrometry. Even measuring the amount of smaller fragments, such as 4 or 6 amino acids in length, may allow to conclude about the amount of surface-exposed marker, if the particular sequence of such fragments is specific for the marker, i.e. there are no other fragments of the same size and sequence in the sample originating from different proteins or peptides, or if the concentration of such different proteins or peptides is negligible or independent from the disorder, dysfunction or disease to be diagnosed.

A suitable protease may be of any kind deemed suitable by the person skilled in the art. Preferably, the protease is site-specific, i.e. it cuts the marker at a known site. However, the protease can also be rather unspecific, as long as reliable measurement of the fragment is possible. An example for a suitable protease may be the protease which naturally cleaves CD40L from the surface of the platelets (see e.g. Otterdal, K., Pedersen, T.M., and Solum, N.O. (2004). Release of soluble CD40 ligand after platelet activation - Studies on the solubilisation phase. Thrombosis Research, vol. 114, pp. 167-177). Examples for suitable proteases are described in US 6,861,504 to Phillips et al. (granted March 1, 2005) in the context of inhibitors of proteases whose target is CD40. Also for other markers suitable proteases can be easily determined by the person skilled in the art also for other markers, for example by means of software listing proteases for a given sequence. Such software, e. g. "PeptideCutter" is available to the person skilled in the art, e.g. via the website www.expasy.org. Many proteases are commerically available and can be easily tested by methods known to the person skilled in the art whether they are suitable for cleaving the respective surface-exposed marker of interest.

Site-specific proteases are known, for example Furin (available from New England Biolabs, Beverly, MA, USA) cuts preferably the sequence Arg-X-X-Arg, but with preference for the site Arg-X-X (Lys/Arg)-Arg.

The enzymatic action of the protease may be stopped by a suitable means, for example furine is inhibited by EGTA, α1-antitrypsine and by polyarginine compounds.

Suitable site-specific proteases can furthermore be identified by assays known to the person skilled in the art (see e.g. Grueninger-Leitch, F., Berndt, P., Langen, H., Nelbock, P. et al. (2000). Identification of β-secretase-like activity using a mass spectrometry-based assay system. Nature Biotechnology, vol. 18, pp. 66-70). The cited method is a high-throughput assay system for detecting site-specific protease from crude cell homogenates. The protein substrate is coupled to ceramic beads and incubated with cell homogenates; then the peptides are washed, released, and analyzed by mass spectrometry, allowing for purification of proteases that clip a protein at the site of interest.

The term "activating" platelets is known to the person skilled in the art. E.g. in the aggregometric methods known in the art, platelets are activated by contacting a sample with an activator of platelets. Particularly, platelet activation may be understood as relating to any method which results in shedding of sCD40L from the CD40L present on the surface of platelets, more particularly any method which results in shedding of a detectable amount, preferably of at least 30%, at least 50%, at least 70%, at least 80%, at least 90%, or at least 95% of the total amount of CD40L present on the platelet surface within less than 10, 8, 7, 5, 3 or 2 minutes. The higher the fraction of surface exposed CD40L which is proteolyzed and liberates sCD40L, and/or the faster the shedding takes place, the higher is the level of activation achieved. Alternatively or additionally, platelet activation may be understood as any method which results in shape changes typical of platelet activation (i.e. actin polymerisation and/or formation of pseudopodia, within less than 20, 10, 8, 7, 5, 3 or 2 minutes. The more pronounced the shape change is and/or the higher the fraction of platelets in the sample which show the shape change and/or the faster the shape change takes place, the higher is the level of activation achieved. Again alternatively or additionally, platelet activation may be understood as any method which results in degranulation of dense granula and/or other granula and/or lysosomes from the platelets who less than 20, 10, 8, 7, 5 or 3 minutes. The more pronounced the degranulation is and/or the higher the fraction of platelets in the sample which show the degranulation and/or the faster the degranulation takes place, the higher is the level of activation achieved.

Preferably, activation is carried out exogenously, i.e. not by an activator already naturally present in the blood sample (such natural activators may include Collagen, Fibrinogen, Ca²⁺, von Willebrand Factor (vWF), Thrombin, serotonin in their naturally in the sample occurring concentrations). For example, a platelet activator may be added to the sample in order to carry out exogenous activation. Of course such exogenously added activator may have the same chemical structure as an activator already present in the sample (e.g. activators such as ADP may also be present as natural activators in the sample). However, activation by a naturally present activator may be considered if this process appears to be sufficiently controllable or standardizable.

The step of activating the platelets can be carried out by any means deemed appropriate, for example mechanically (e.g. by stirring the sample or by passing the sample through a fleece), electrically (e.g. by applying voltage to the sample), thermically (e.g. by applying heat to the sample), or chemically (e.g. by a platelet activator). The methods may also be combined, e.g. the fibers of a fleece can be coated with a platelet activator. Means for activating platelets, particularly platelet activators, can be found by their ability of induce platelet activation as described elsewhere in this specification. Platelet activation can be measured quite easily according to methods known in the art, see elsewhere in this specifcation. For example, a platelet activator can be found by contacting a blood sample with a candidate for a platelet activator and measuring the time until activation occurs, e.g. a shape change, or by measuring the extent of shedding of sCD40L from the surface of platelets. If the candidate reduces the time until activation in such a test compared to activation in a control sample not contacted with the candidate, then the candidate is identified as being a platelet activator. Similarly, if the candidate increases the amount of sCD40L shedded from the platelet surface or if such candidate reduces the time required for such shedding, then the candidate is identified as being a platelet activator.

Preferably, the platelet activator is not an activator of the plasmatic coagulation system ("a coagulation activator"). Such coagulation activators are occasionally added to blood samples in order to enhance blood coagulation. By releasing endogenous platelet activating agents from the sample, such coagulation activators may indirectly lead to an activation of blood platelets.

Examples for suitable platelet activators are known and include e.g. collagen, ADP, epinephrin, arachidonic acid, ristocetin, FIIa (thrombin), TRAP (thrombin-receptor activation peptide; the peptide sequence is SFLLRN) thromboxane A₂, PAF (platelet-activating factor), GPRP (the peptide gly-pro-arg-pro), serotonin, dopamine, collagen-related peptide, U-46619, and any synthetic analogues of said molecules, etc. The choice of the activator may also be made according to the suspected disorder of the patient. For example, in the case of suspected Bernard-Soulier syndrome, ristocetin may be a preferable activator.

Activation is preferably carried out in a standardizable and/or controlled manner, e.g. by adding a platelet activator in a predetermined concentration, to the sample, for example 1-100, preferably 5-20 µM ADP, or 0.5-20, preferably 1-5 µg/ml collagen.

The activator may already be present in a container into which the blood sample is to be added or, preferably, in a blood sampling device used for taking the blood sample. In this case, the activator may get immediately into contact with the blood sample upon addition of the sample.

In one embodiment, the invention relates to diagnosing a platelet stimulation capability (PSC). In this case, the marker of platelet function is brought into solution according to any method laid out in this specification (preferably by activating the platelets in the sample) and the activation and/or the step of bringing the marker of platelet function into solution is stopped (e.g. by centrifugation) after a particular time period. Preferably the time period is shorter than required to reach a steady state of the released level of the marker of platelet function. An aim of this method is to obtain a parameter of how quickly the marker of platelet function is released, particularly upon activation. The resulting sample is preferably centrifuged in order to obtain a supernatant containing the marker of platelet function. The supernatant may be separated from the pellet containing the platelets and possibly other blood cells. This would avoid further leakage of the marker of platelet activation from the platelets into the sample. However, depending on the force of centrifugation and the further handling, the pellet can be sufficiently stable to obtain a stable plasma sample also if the supernatant is not separated. The level of the marker of platelet function can then be measured in the supernatant.

The step of bringing the marker of platelet function into solution (e.g. activation of the platelets) can be stopped by any means deemed appropriate e.g. by separating the platelets from the plasma, (e.g. by centrifugation and/or filtration), or by adding an inhibitor of Glycoprotein/IIb/IIIa (such as tirofiban, eptifibatide or Abciximab), an inhibitior of metalloproteinases (such as disclosed in US 6,861,504 to Phillips et al., cited above) EDTA, cytochalasin D, formaldehyde, inhibiting ("blocking") antibodies against CD40L (e.g. the blocking antibody G28-5, available from the American Type Culture Collection (ATCC), ATCC No. HB-9110, see Otterdal, K. et al., cited above), or any other inhibitors of platelet activation (e.g., methyl-S-adenosine mono-phosphate (Me-S-AMP), aspirin, or terbogrel).

In another embodiment, the present invention relates to diagnosing a global functional platelet capacity (GFPC). In this case, the marker of platelet function is brought into solution according to any method laid out in this specification (preferably by activating the platelets in the sample) and the step of bringing the marker of platelet function into solution is stopped (e.g. by centrifugation) after a time-period which is sufficient to release almost the total amount of the marker of platelet function from the surface of the platelets. An aim of this method is to obtain a parameter of the total level of platelet stimulation in vivo. A suitable time-period can be e.g. the time required until the released level of the marker of platelet function reaches a steady state or almost a steady state. Further centrifugation or processing can be carried out as described for PSC.

Preferably, the platelets in the sample are activated without fibrin formation and/or coagulation taking place before and/or during activation of the platelets. Fibrin is formed from fibrinogen. For example, if a blood sample is left untreated at room temperature, fibrinogen will be transformed into fibrin. Excessive fibrin formation should be avoided, because the fibrin meshwork may interfere with a subsequent solubilisation of the marker of platelet function.

Coagulation is a process which results in one or more fibrin clots and/or one or more blood clots. In the process of fibrin formation or coagulation, platelets may (1) either get activated by intermediate products of the coagulation process, and/or (2) platelets may get caught inside of a blood clot or fibrin meshwork. In the first case, the coagulation process may result in additional release of a marker of platelet function (e.g. shedding of sCD40L) and thus result in a higher level of the marker of platelet function and/or in faster release of the marker of platelet function. In the second case, not all platelets may get equally activated (e.g. due to insufficient exposure to a platelet activator), thus resulting in a lower level of the marker of platelet function and/or in slower release of the marker of platelet function. In any of both cases, excessive coagulation may distort the result of measurement. Thus, excessive fibrin formation or coagulation is preferably avoided. The person skilled in the art is familiar with determining a level of fibrin formation or and/or coagulation which is still tolerable and/or with determining a level of fibrin formation and/or coagulation which is excessive. In general, a degree of fibrin formation and/or coagulation which does not impair the diagnostic value of the result is still tolerable. On the other hand, a degree of fibrin formation and/or coagulation which results in false positive or false negative diagnoses, may be excessive and is preferably avoided.

Preferably, fibrin formation and/or coagulation is avoided until the marker of platelet function is measured or has been measured. However, if the activation of the platelets is stopped for example by centrifugation before measuring of the marker of platelet function, coagulation or fibrin formation in the pellet may be tolerable, as it will not lead to excessive additional leakage of a marker of platelet function into the supernatant. Then, the marker of platelet function can be measured in the supernatant without impaired diagnostic value.

Therefore, the sample is preferably kept under conditions capable of inhibiting fibrin formation or coagulation. Such conditions may include diluting the sample (so that no relevant fibrin meshwork can form) or keeping the sample on ice, (in this case, the sample is preferably warmed up to room temperature once the platelets are activated or a protease is added) or contacting the sample with an anticoagulant. The person skilled in the art is familiar with determining whether an agent is able to act as an anticoagulant. Any agent capable of inhibiting fibrin formation and/or coagulation may be understood as being an anticoagulant. Inhibition of fibrin formation or coagulation can be measured quite easily according to methods known in the art, see elsewhere in this specification. For example, an anticoagulant can be found by contacting a blood sample with a candidate for an anticoagulant and measuring the time until fibrin formation or coagulation occurs, e.g. until blood clots are formed or until a given percentage of fibrinogen is processed into fibrin. If the candidate increases the time until fibrin formation and/or coagulation takes place in such a test compared to fibrin formation and/or coagulation in a control sample not contacted with the candidate, then the candidate is identified as being an anticoagulant.

Examples for suitable anticoagulants are known and include agents which are capable of binding calcium ions (such as citrate or ethylenediaminetetraacetate (EDTA)). Such agents may bind calcium ions with different affinities. E.g. citrate binds calcium ions with relatively low affinity whereas EDTA binds with relatively high affinity. Higher affinity has the advantage that it shows better inhibition of coagulation, but may require calcium to be added e.g. during measurement of the marker of platelet function, if such measurement should require calcium. Lower affinity may result in less inhibition, but may be better tolerated during further downstream processing.

Further examples for anticoagulants include indirect or direct thrombin inhibitors, such as heparin (including unfractionated heparin as well as low molecular weight heparin (e.g. Fraxiparin), heparinoids (e.g. Danaproid-Natrium), hirudin (including synthetic derivatives thereof, such as Lepirudin or Desirudin), hirudin analoga (e. g. bivalirudin, argotroban, melagatran) or D-phenylalanyl-L-propyl-L-arginine chloromethyl ketone (PPACK).

Preferred anticoagulants are agents which do not act by binding of calcium ions. This may be advantageous if calcium ions are required during further processing of the sample.

The invention takes advantage of certain markers. The level of a suitable marker can indicate the presence or absence of the condition, disorder, dysfunction, disease, risk or complication, and thus allow diagnosis. More particularly, the present invention relates to biochemical markers. The term "biochemical marker" is known to the person skilled in the art. In particular, a biochemical marker is a gene expression product (e.g. protein or peptide) which is differentially expressed (i.e. upregulated or downregulated) in presence or absence of a certain condition, disorder, dysfunction, disease, risk or complication.

Markers of platelet function are known to the person skilled in the art. In the sense of the invention, any agent, particularly any protein or peptide, which is differentially released from and/or expressed on the surface of the platelets stimulated in vivo compared to platelets which have not been stimulated in vivo can be understood as a suitable marker of platelet function. Particularly, the marker is a marker which is present in the granular vesicles of the platelets and more strongly expressed on the platelet surface after stimulation in vivo.

The granular vesicles are organelles of the platelets and fuse with the platelet membrane as part of the so-called "basic platelet reaction" after stimulation in vivo.

The person skilled in the art is able to identify such markers. For example, samples of platelets which have been stimulated in vivo can be compared with samples of platelets which have not been stimulated in vivo (a certain degree of in vivo stimulation can be induced e.g. by a fat- and protein-rich meal; stimulation in vivo also occurs in the course of certain cardiovascular disorders, such as described elsewhere in the specification). The platelets can then be sorted e.g. by FACS according to the level of surface expression of CD40L (see e.g. Mason et al., cited above) and then the sorted platelets can be analyzed with respect of the surface expression of other molecules, e.g. by using an antibody library. Any protein or peptide which is co-expressed with CD40L (i.e. any marker which is also differentially expressed upon stimulation in a similar manner to sCD40L) can then be considered to be a suitable marker. The person skilled in the art will appreciate that essentially any agent contained in the granular vesicles or, particularly, in a membrane of the granular vesicles is also a good candidate for a suitable marker according to the present invention.

Examples for suitable markers include, but are not limited to CD40L, sCD40L, P-selectin (CD62), glycoprotein IV (GP-IV), glycoprotein V (GP-V), glycoprotein VI (GP-VI, also known as p62), CD63(granulophysin), β-thromboglobulin (β-TG), platelet factor 4 (PF-4). Preferably, the marker is a membrane-bound marker, which can be shed from the platelet surface e.g. by activation in vitro and/or by action of a protease. Such markers include, but are not limited to CD40L, P-selectin (CD62), GP-IV, GP-V, GP-VI. The respective fragments which are physiologically shedded by activation of the platelets are designated sCD40L (soluble CD40L), soluble P-selectin, soluble GP-IV, soluble GP-V, soluble GP-VI. In contrast, β-TG and PF-4 are soluble molecules contained in the granular vesicles, which are released from the platelets upon stimulation or activation. CD63 (granulophysin) is contained in lysosomal vesicles and is released from the platelets upon stimulation or activation. In summary, examples for suitable markers include CD40L, sCD40L, P-selectin, soluble P-selectin, GP-IV, soluble GP-IV, GP-V, soluble GP-V, GP-VI, soluble GP-VI, CD63, platelet factor-4 (PF-4), β-Thromboglobulin (β-TG), or any variants thereof.

The term "variants" in this context relates to proteins or peptides substantially similar to said proteins or peptides. The term "substantially similar" is well understood by the person skilled in the art. In particular, a variant may be an isoform or allele which shows amino acid exchanges compared to the amino acid sequence of the most prevalent isoform in the human population. Preferably, such a substantially similar protein or peptide has a sequence similarity to the most prevalent isoform of the protein or peptide of at least 80%, preferably at least 85%, more preferably at least 90%, most preferably at least 95%. Substantially similar are also fragments or degradation products, e.g. proteolytic fragments or degradation products, which are still recognized by the diagnostic means or by ligands directed against the respective full-length protein or peptide. In this context, it is expressly referred also to the description and definitions of suitable fragments given in the context of bringing the marker of platelet function into solution by means of proteolysis, see abstract. The term "variants" is also meant to relate to splice variants.

The term "variant" also relates to a post-translationally modified protein or peptide such as glycosylated peptide. A "variant" is also a protein or peptide which has been modified after collection of the sample, for example by covalent or non-covalent attachment of a label, particularly a radioactive or fluorescent label, to the protein or peptide.

Examples of particular variants and methods for their measurement are known. For example, there are different variants of sCD40L (see e.g. Pietravalle, F. et al. (1996). Human Native Soluble CD40L is a Biologically Active Trimer, Processed Inside Microsomes. J Biol Chem, vol. 271, pp. 5965-7).

Other embodiments of the invention include the measuring of different markers of platelet function in combination, simultaneously or non-simultaneously.

The invention is of particular use in the field of diagnosis and consequently also relates to methods of diagnosing. Diagnosing according to the present invention includes determining, monitoring, confirmation, subclassification and prediction of the relevant disease, disorder, complication, or risk. Determining relates to becoming aware of a disease, disorder, complication, or risk. Monitoring relates to keeping track of an already diagnosed disease, disorder, or complication, e.g. to analyze the progression of the disease or the influence of a particular treatment on the progression of disease or complication. Confirmation relates to the strengthening or substantiating a diagnosis already performed using other indicators or markers. Subclassification relates to further defining a diagnosis according to different subclasses, e.g. defining according to mild and severe forms of a disease. Prediction relates to prognosing a disease, disorder or complication or risk before other symptoms or markers have become evident or have become significantly altered.

According to the invention, the term diagnosing shall also be understood as relating to determining a certain function, dysfunction, degree of stimulation or any other relevant parameter of interest in the context of the invention (e.g. platelet stimulation capability or global functional platelet capacity).

Diagnosis can be improved, if the result of the measurement of the marker is put into relation to the number of platelets present in the sample, e.g. if the concentration of sCD40 is divided by the number of platelets present in the sample.

Typically, final diagnosis will be carried out by a physician who also takes into account e.g. the disease history of the patient. Thus, the invention may provide a diagnostic parameter. Consequently, the invention also relates to a method of obtaining or generating a diagnostic parameter.

The person skilled in the art is familiar with methods of measuring the number of platelets present in the sample, e.g. by an automated counter (e.g. Coulter counter, Sysmex Inc.) or by manual blood cell counting by use of a microscope.

The present invention is e.g. relevant for diagnosing platelet function and dysfunction. The invention is also relevant for diagnosing any kind of dysfunction in which platelets are stimulated in vivo.

Diagnosis of platelet function is e.g. important in the quality control of blood donations, particularly platelet donations, and platelet preparations, (including any platelet packs, concentrates, or products). The invention allows for example to determine whether the platelets in such a donation or preparation are fully functional and can be used for platelet substitution, e. g. to reduce the risk of bleeding in a subject. Such subject may e.g. be a patient suffering from thrombocytopenia (insufficient number of platelets). Those patients frequently receive platelet transfusions either on a regular basis or before operations involving a risk of bleeding.

An abnormal level, particularly a decreased level, of the solubilized marker of platelet function as measured according to the invention may indicate that said donation and/or platelet preparation may not be of sufficient quality for platelet substitution, e. g it may not be able to sufficiently reduce the risk of bleeding.

An abnormal level, particularly an increased level, of the solubilized marker of platelet function as measured according to the invention may indicate an increased risk of transfusion-related acute lung injury (TRALI).

Diagnosis of platelet function is also important in the context of the control of therapy with inhibitors of platelet function, e.g. therapy with at least one agent chosen from the group consisting of acetylsalicylic acid, inhibitors of platelet ADP receptors, phosphodiesterase inhibitors and inhibitors of GpIIb/IIIa (e.g. Abciximab, lanifiban, roxifiban, tirofiban). Examples of such agents include thienopyridines (e.g. ticlopidine and clopidogrel). Therapy with inhibitors of platelet function is frequently carried out in patients suffering from cardiovascular dysfunctions, e.g. in the case of coronary heart disease (including all subforms of coronary heart disease as described elsewhere in this specification, particularly acute coronary syndrome, more particularly myocardial infarction), after stent implantation, after balloon dilatation, or after artificial heart valve implantation.

Platelet dysfunctions may include platelet hypofunction as well as platelet hyperfunction.

Examples for platelet hypofunction include hemorrhagic diathesis associated with dysfunction of platelets, inherited deficiencies of platelet surface glycoproteins, dense granule storage pool diseases, and platelet hypofunction observed in very-low-birth-weight preterm neonates (such neonates show a propensity to intraventricular hemorrhage). Further examples for platelet hypo- or hyperfunction and diseases associated with platelet hypo- and hyperfunction are mentioned e.g. in Hayward CPM et al. as well as in Michelson AD and Furman Ml., both of which are incorporated herein by reference, particularly any examples for platelet hypo- and hyperfunction and associated diseases mentioned therein (Hayward CP et al., Congenital platelet disorders: overview of their mechanisms, diagnostic evaluation and treatment. Haemophilia 2006, 12 (suppl. 3): 128-36); Michelson AD and Furman Ml., Laboratory markers of platelet activation and their clinical significance. Curr Opin Hematol 1999, 6: 342-8.)

Diagnosis of platelet dysfunctions may also be carried out in the context of screening subjects for the presence of a platelet dysfunction. Such diagnosis of platelet dysfunction would allow to identify subjects at risk of suffering from increased or excessive bleeding as a consequence of a platelet dysfunction. Particularly, such diagnosis may be carried out before a planned surgery. Such methods would allow to identify subjects who may preferably be treated for the dysfunction before surgery, e.g. by administering a platelet transfusion. Therefore, the present invention also relates to a method of deciding about administering a platelet transfusion before planned surgery. The term "increased bleeding risk" in this context is understood by the person skilled in the art and preferably relates to a risk of bleeding which is increased as compared to an average subject or patient.

The invention also allows diagnosis of any disorder in which an increased or decreased level of CD40L on the surface of the platelet membrane can be measured. The term "increased or decreased level" in this context relates to a level (i.e. concentration or amount) which is increased or decreased as compared to an average comparable subject (e.g. of the same age and gender) which does not suffer from the disorder. More particularly, the term "increased or decreased level" relates to a level which is increased or decreased in the same individual compared between before the disorder and in presence of the disorder. Such diseases can be identified by the person skilled in the art, e.g. by measuring the level of sCD40L according to one of the methods of the invention, preferably in the context of clinical studies. More particularly, good candidates for such disorders and clinical studies are disorders which have already been discussed in the literature as being associated with increased or decreased levels of sCD40L.

Examples for disorders which have already been discussed in the literature as being associated with increased or decreased levels of sCD40L include the following: cardiovascular risk factors (e.g. morbid obesity, essential hypertension, diabetes mellitus, endothelial function, familiar hypercholesterolemia, obstructive sleep apnea syndrome); cardiovascular disease (e.g. early onset occlusive carotid artery disease, prediction of restenosis after percutaneous coronary intervention, coronary and cerebral vascular disease, advanced symptomatic peripheral arterial diseases, hypertensive heart disease, acute and chronic heart failure, pulmonary arterial hypertension, Kawasaki disease); pharmalogical resistance/drug efficacy (e.g. aspirin failure); inflammation and immunological disorders (e.g. systemic autoimmune diseases, lupus erythematosus, systemic sclerosis, inflammatory bowel disease, mixed connective tissue disease); other diseases associated with platelet dysfunction or activation (e.g. preeclampsia, acute severe pancreatitis, cystic fibrosis, lung cancer).

As already mentioned, the invention also allows diagnosis of disorders in which platelets are stimulated in vivo. Such disorders can be identified by the person skilled in the art, e.g. by means of aggregometry. Disorders in which platelets are stimulated in vivo are also known to the person skilled in the art. Examples include thrombophilia, cardiovascular disorders, particularly cardiovascular disorders associated with inflammation and/or plaque formation and/or plaque rupture and/or thrombus formation and/or any other disorders characterized by a high thromboembolic risk.

Cardiovascular disorders associated with plaque formation and/or plaque rupture, and/or thrombus formation the cardiovascular system are widely known. A plaque or thrombus may cause a narrowing or closure of a blood vessel and thus reduce blood flow and oxygen supply. Frequently, the clinically relevant disorder is caused by a ruptured plaque or by a thrombus which is carried along with the blood stream until it occludes a blood vessel, such as a coronary or pulmonary artery.

In the cardiac system (heart and coronary arteries), disorders associated with plaque or thrombus formation may include stable angina pectoris (SAP) and acute coronary syndromes (ACS). Angina pectoris is characterized by myocardial ischemia, i.e. insufficient of oxygen supply, particularly under conditions of increased oxygen demand, e.g. in the case of physical exercise. ACS patients can show unstable angina pectoris (UAP), or these individuals have already suffered from a myocardial infarction (MI). Myocardial infarction is characterized by small or larger regions of necrosis (i.e. tissue destruction due to lack of oxygen supply). According to the appearance in the electrocardiogram, MI can be an ST-elevation MI or a non-ST-elevation MI. The occurring of an MI can be followed by a left ventricular dysfunction (LVD). LVD patients develop congestive heart failure (CHF) with a mortality rate of roughly 15 %.

However, disorders associated with plaque or thrombus formation may also occur in peripheral vessels and result e.g. in gangrene, microangiopathy, or pulmonary embolism.

In the context of the present invention, "cardiovascular disorder" particularly relates to coronary heart disease, SAP, ACS, UAP, MI, ST-elevation MI, non-ST-elevation MI, LVD, CHF, or cardiovascular death. More particularly, "cardiovascular disorder" relates to ACS, UAP, MI, ST-elevation MI, non-ST-elevation MI, LVD, or CHF.

Cardiovascular disorders can cause symptoms, which have been classified into a functional classification system according to the New York Heart Association (NYHA). Patients of Class I have no obvious symptoms of cardiovascular disorder. Physical activity is not limited, and ordinary physical activity does not cause undue fatigue, palpitation, or dyspnea (shortness of breath). Patients of class II have slight limitation of physical activity. They are comfortable at rest, but ordinary physical activity results in fatigue, palpitation, or dyspnea. Patients of class III show a marked limitation of physical activity. They are comfortable at rest, but less than ordinary activity causes fatigue, palpitation, or dyspnea. Patients of class IV are unable to carry out any physical activity without discomfort. They show symptoms of cardiac insufficiency at rest. If any physical activity is undertaken, discomfort is increased.

Another functional classification is the grading of angina pectoris by the Canadian Cardiovascular Society (CCS) classification system:
Class I: Ordinary physical activity does not cause angina, such as walking, climbing stairs. Angina [occurs] with strenuous, rapid, or prolonged exertion at work or recreation.
Class II: Slight limitation of ordinary activity. Angina occurs on walking or climbing stairs rapidly, walking uphill, walking or stair climbing after meals, or in cold, or in wind, or under emotional stress, or only during the few hours after awakening. Walking more than two blocks on the level and climbing more than one flight of ordinary stairs at a normal pace and in normal condition.
Class III: Marked limitations of ordinary physical activity. Angina occurs on walking one to two blocks on the level and climbing one flight of stairs in normal conditions and at a normal pace.
Class IV: Inability to carry on any physical activity without discomfort -- angina symptoms may be present at rest.

Accordingly, patients suffering from cardiovascular disorders can be divided into individuals showing no clinical symptoms and those with symptoms (e.g. dyspnea).

Another characteristic of cardiovascular disorders can be the "left ventricular ejection fraction" (LVEF) which is also known as "ejection fraction". People with a healthy heart usually have an unimpaired LVEF, which is generally described as above 50 %. Most people with a systolic heart disease which is symptomatic generally have an LVEF of 40 % or less.

A cardiovascular disorder according to the invention may cause symptoms, particularly symptoms according to NYHA class II-IV, more particularly according to NYHA class III-IV. The cardiovascular disorder may also cause symptoms according to the CCS classification, particularly according to CCS class II-IV, more particularly according to CCS class III-IV.

A cardiovascular disorder according to the invention may be associated with an LVEF of 40% or less.

A cardiovascular disorder according to the invention may either be "compensated" or "decompensated". Compensated means that the regular oxygen need of the body can still be satisfied, whereas decompensated means that the regular oxygen need of the body is not satisfied anymore.

Another use of the invention relates to diagnosing the risk of a subject of suffering from a disorder chosen from the group consisting of HIT (heparin-induced thrombocytopenia), thrombocytopenic thrombotic purpura, HELLP syndrome (hemolysis, elevated liver enzymes, and low platelet count), and thrombotic microangiopathy. HIT can be divided into two groups, HIT type 1 (the less severe form) and HIT type 2 (the more severe form with a lethality of about 25%). Particularly, the present invention also allows to diagnose the risk of suffering from HIT type 2. In diagnosis of risk, the present invention allows diagnosis of the risk also in subjects not presently suffering from the relevant disorder.

Yet another use of the invention relates to diagnosing thrombophilia, particularly in patients with deficiencies of coagulation inhibitors, e. g. Antithrombin, Protein C, Protein S, or Activated-Protein-C resistance (APC resistance, e.g. Factor V-Leiden) and prothrombin polymorphism (e.g. G2021A) and to diagnosing hypercoagulability.

The invention also relates to monitoring platelet function, platelet dysfunction, or a disorder in which platelets are stimulated, or any other diagnoses or uses as laid out in this specification.

The term "monitoring" is known to the person skilled in the art and has been defined elsewhere in this specification. More particularly, monitoring may be carried out by diagnosing the risk of the patient in regular intervals, for example at intervals of approximately 2 hours, 10 hours, 1 day, 2 days, 3 days, 4 days, 1 week, 2 weeks, 3 weeks, 4 weeks, 1 month, , 2 months, 4 months, 6 months, or 1 year. The term "approximately" in this context is understood by the person skilled in the art. Therefore, the actual interval may also deviate from an intended regular interval depending on practical circumstances such as arranging for suitable appointments etc.. For example, the interval may deviate by up to 100%, preferably up to 50%, more preferably up to 25%, more preferably up to 10%.

The term "subject" according to the present invention relates to a healthy individual, an apparently healthy individual, or a patient. The term "patient" particularly relates to an individual suffering from a disease or disorder as described elsewhere in this specification. More particularly, the patient is suffering from or treated for a platelet dysfunction or a cardiovascular disorder as described elsewhere in this specification.

Diagnosis according to the present invention is preferably carried out by use of a diagnostic means. A diagnostic means is any means that allows to measure the level, amount, or concentration of a substance of interest, particularly a peptide, polypeptide, or protein of interest, more particularly a marker of platelet function.

Methods and diagnostic means which can be used to determine the levels of the respective peptides, polypeptides, or proteins are known to the person skilled in the art. These methods include microplate ELISA-based methods, fully-automated or robotic immunoassays (available for example on Elecsys^{™} or Cobas^{™} analyzers), CBA (an enzymatic Cobalt Binding Assay, available for example on Roche-Hitachi^{™} analyzers), and latex agglutination assays (available for example on Roche-Hitachi^{™} analyzers). The methods and means for measurement also include Point-of-care devices, such as the Cardiac Reader^{™} (available from Roche Diagnostics).

Point-of-care devices are generally understood as devices which enable measuring at the patient bedside. An example is the Cardiac Reader^{™} (available from Roche Diagnostics), in combination e.g. with test strips for NT-proBNP (available as "Cardiac proBNP" from Roche Diagnostics). Such test may employ two (preferably monoclonal) antibodies directed against the peptide of interest (e.g. a BNP-type peptide). The antibodies may be identical to the antibodies used e.g. in the Elecsys^{™} or Cobas^{™} assays. E.g. the first antibody is labeled with biotin while the second antibody is labeled with gold particles. The test can be started by adding a small amount (e.g. 150 µl) of blood sample onto the test strip (e.g. into a sample well of the test strip). The erythrocytes in the sample may be separated from the remaining plasma before or after addition to the test strip, e.g. if the sample flows through a suitable fleece (e.g. a glass fiber fleece). Said separating means (e.g. fleece) is preferably part of the test strip. The antibodies (preferably already present on the test strip) are dissolved in the remaining plasma. The antibodies are capable of binding to the peptide or polypeptide of interest, forming a three-membered sandwich complex. The antibodies (bound or unbound) flow through the strip into a detection zone. The detection zone comprises means for detecting the bound complex, e.g. it may comprise streptavidin. This immobilizes the complexes and visualizes the immobilized complex as a purple line by the gold-labeled antibody. Preferably, remaining free gold-labeled antibody may then move further down the strip where it is captured in a zone comprising a synthetic peptide or polypeptide comprising the epitope of the BNP-type peptide to be detected, visualized as a separate purple line. The presence of such second line can serve as a control because it indicates that the sample flow as worked correctly and the antibody is intact. the test strip may comprise a label indicating which peptide or polypeptide of interest can be detected with the strip. It may also comprise a barcode or other code readable by a device for optical measurement of the amount of label detectable in the detection zone. Such barcode may include information indicating which peptide or polypeptide of interest can be detected with the strip. The barcode may also include lot-spcific information about the test strip.

The Cardiac Reader itself comprises a camera (e.g. charge-coupled device, CCD) that optically records the detection zone of the test strip. Signal and control lines may be identified by a pattern recognition algorithm. The intensity of the label in the signal line is typically proportional to the amount of peptide or polypeptide of interest. The optical signal may be converted into a concentration via a lot-specific calibration curve which may be stored in a code chip. The agreement of calibration code and test lot may be checked by a barcode on the test strip.

Furthermore, the person skilled in the art is familiar with different methods of measuring the level of a peptide, polypeptide, or protein. The term "level" relates to amount or concentration of a peptide or polypeptide in a patient or a sample taken from a patient.

The term "measuring" according to the present invention relates to determining the amount or concentration, preferably semi-quantitatively or quantitatively, of the nucleic acid, peptide, polypeptide, or protein, or other substance of interest. Measuring can be done directly or indirectly. Indirect measuring includes measuring of cellular responses, bound ligands, labels, or enzymatic reaction products.

In the context of the invention, amount also relates to concentration. It is evident, that from the total amount of a substance of interest in a sample of known size, the concentration of the substance can be calculated, and vice versa.

Measuring can be done according to any method known in the art. Preferred methods are described in the following.

In a preferred embodiment, the method for measuring the level of a peptide, polypeptide, or protein of interest, comprises the steps of (a) contacting a cell capable of a cellular response to the peptide or polypeptide with the peptide or polypeptide for an adequate period of time, (b) measuring the cellular response.

In another preferred embodiment, the method for measuring the level of a peptide, polypeptide, or protein of interest, comprises the steps of (a) contacting a peptide or polypeptide with a suitable substrate for an adequate period of time, (b) measuring the amount of product.

In another preferred embodiment, the method for measuring the level of a peptide, polypeptide, or protein of interest, comprises the steps of (a) contacting a peptide or polypeptide with a specifically binding ligand, (b) (optionally) removing non-bound ligand, (c) measuring the amount of bound ligand.

Preferably, the peptide, polypeptide, or protein is contained in a sample, particularly a body fluid or tissue sample, and the amount of the peptide, polypeptide, or protein in the sample is measured.

Peptides, polypeptides, or proteins can be measured in any sample according to the invention.

If necessary, the samples may be further processed before measuring the peptide, polypeptide or protein. Particularly, nucleic acids, peptides or polypeptides may be purified from the sample according to methods known in the art, including filtration, centrifugation, or extraction methods such as chloroform/phenol extraction.

For measuring cellular responses, the sample or processed sample is added to a cell culture and an internal or external cellular response is measured. The cellular response may include the expression of a reporter gene or the secretion of a substance, e.g. a peptide, polypeptide, protein or a small molecule.

Other preferred methods for measurement may include measuring the amount of a ligand binding specifically to the peptide, polypeptide, or protein of interest. Binding according to the present invention includes both covalent and non-covalent binding.

A ligand according to the present invention can be any peptide, polypeptide, nucleic acid, or other substance binding to the peptide or polypeptide, or protein of interest. It is well known that peptides, polypeptides, or proteins, if obtained or purified from human or animal cells, can be modified, e.g. by glycosylation. A suitable ligand according to the present invention may bind the peptide, polypeptide, or protein also via such sites.

Preferably, the ligand should bind specifically to the peptide, polypeptide, or protein to be measured. "Specific binding" according to the present invention means that the ligand should not bind substantially to ("cross-react" with) another peptide, polypeptide, protein, or substance present in the sample investigated. Preferably, the specifically bound protein or isoform should be bound with at least 3 times higher, more preferably at least 10 times higher and even more preferably at least 50 times higher affinity than any other relevant peptide, polypeptide, or protein. In the present context, such other relevant peptides, polypeptides, or proteins may be other structurally related or homologous peptides, polypeptides, or proteins.

Non-specific binding may be tolerable, particularly if the investigated peptide, polypeptide, or protein can still be distinguished and measured unequivocally, e.g. according to its size on a Western Blot or by its relatively higher abundance in the sample.

Binding of the ligand can be measured by any method known in the art. Preferably, the method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide, polypeptide, or protein of interest, an enzymatic reaction product may be measured (e.g. the amount of a protease can be measured by measuring the amount of cleaved substrate, e.g. on a Western Blot).

For measurement of enzymatic reaction products, preferably the amount of substrate is saturating. The substrate may also be labeled with an detectable lable prior to the reaction. Preferably, the sample is contacted with the substrate for an adequate period of time. An adequate period of time refers to the time necessary for an detectable, preferably measurable amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand.

Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.)

The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus.

Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels.

Enzymatically active labels include e.g. horseradish peroxidase, alkaline phosphatase, beta-Galactosidase, Luciferase, and derivatives thereof Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star^{™} (Amersham Biosciences), ECF^{™} (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously.

Typical fluorescent labels include fluorescent proteins (such as GFP, YFP, RFP and derivatives thereof), Cy3, Cy5, Texas Red, Fluorescein, the Alexa dyes (e.g. Alexa 568), and quantum dots. Further fluorescent labels are available e.g. from Molcular Probes (Oregon).

Typical radioactive labels include ³⁵S, ¹²⁵I, ³²P, ³³P and the like. A radioactive label can be detected by any method known and appropriate, e.g. a light-sensitive film or a phosphor imager.

Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, solid phase immune tests, and mass spectrometry such as SELDI-TOF, MALDI-TOF, or capillary electrophoresis-mass spectrometry (CE-MS). Further methods known in the art (such as gel electrophoresis, 2D gel electrophoresis, SDS polyacrylamid gel electrophoresis (SDS-PAGE), Western Blotting), can be used alone or in combination with labeling or other dectection methods as described above.

Preferred ligands include antibodies, nucleic acids, peptides, polypeptides, proteins, and aptamers, e.g. nucleic acid or peptide aptamers (e.g. spiegelmers or anticalins). Methods to obtain such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display.

The term "antibody" as used herein includes both polyclonal and monoclonal antibodies, as well as fragments thereof, such as Fv, Fab and F(ab)₂ fragments that are capable of binding antigen or hapten.

Suitable antibodies are available e. g. from R&D systems, Bender MedSystems and Roche Diagnostics. E. g. in the case of P-selectin: Available from Bender MedSystems, including Biotin- or FITC-labeled antibodies and available from QED Bioscience Inc. ("clone AK-6). In the case of CD40L: Available from Antigenix America Inc. (including FITC-labeled antibodies), BD Biosciences Pharmingen, Bender MedSystems and others. In summary, suitable polyclonal, monoclonal, labeled- or unlabeled antibodies are widely available and can be obtained from commercial sources. E. g. the website www.biocompare.com provides an overview over available antibodies.

Further antibodies can be easily generated by the person skilled in the art according to well-known methods.

In another preferred embodiment, the ligand, preferably chosen from the group consisting of nucleic acids, peptides, polypeptides, or proteins, more preferably from the group consisting of nucleic acids, antibodies, or aptamers, is present on an array.

Said array contains at least one additional ligand, which may be directed against a peptide, polypeptide, protein or a nucleic acid of interest. Said additional ligand may also be directed against a peptide, polypeptide or a nucleic acid of no particular interest in the context of the present invention. Preferably, ligands for at least three, preferably at least five, more preferably at least eight peptides, polypeptides or proteins of interest in the context of the present invention are contained on the array.

According to the present invention, the term "array" refers to a solid-phase or gel-like carrier upon which at least two compounds are attached or bound in one-, two- or three-dimensional arrangement. Such arrays (including "gene chips", "protein chips", antibody arrays and the like) are generally known to the person skilled in the art and typically generated on glass microscope slides, specially coated glass slides such as polycation-, nitrocellulose- or biotin-coated slides, cover slips, and membranes such as, for example, membranes based on nitrocellulose or nylon.

The array may include a bound ligand or at least two cells expressing each at least one ligand.

It is also contemplated to use "suspension arrays" as arrays according to the present invention (Nolan JP, Sklar LA. (2002). Suspension array technology: evolution of the flat-array paradigm. Trends Biotechnol. 20(1):9-12). In such suspension arrays, the carrier, e.g. a microbead or microsphere, is present in suspension. The array consists of different microbeads or microspheres, possibly labeled, carrying different ligands.

The invention further relates to a method of producing arrays as defined above, wherein at least one ligand is bound to the carrier material in addition to other ligands.

Methods of producing such arrays, for example based on solid-phase chemistry and photo-labile protective groups, are generally known (US 5,744,305). Such arrays can also be brought into contact with substances or substance libraries and tested for interaction, for example for binding or change of confirmation. Therefore, arrays comprising a peptide, polypeptide, or protein as defined above may be used for identifying ligands binding specifically to said peptides, polypeptides or proteins.

The method according to the invention may also comprise the step of diagnosing platelet function, platelet dysfunction, or a dysfunction in which platelets are stimulated, or any other diagnoses or uses as laid out in this specification, by comparing the measured level of the marker of platelet function to a reference level associated with a particular diagnosis.

The term "reference-level" is known to the persons skilled in the art. Particularly, a reference-level may be associated with a particular diagnosis or it may distinguish between different kinds of diagnosis (e. g. disorder present vs. disorder absent). It will be appreciated that the reference level may also be chosen according to the desired sensitivity or a specificity of the diagnosis. A higher sensitivity means that a higher fraction of a patients having a particular disorder are identified and/or that less patients with a particular disorder will be diagnosed as not having the diagnosed disorder. A higher specificity means that a higher fraction of the patients identified as having a particular disorder do indeed have the diagnosed disorder. The higher desired sensitivity for a particular disorder, the lower is the specificity of the diagnosis and vice versa. Therefore, the reference level may be chosen by the persons skilled in the art according to the desired sensitivity and specificity. Means for determining suitable reference levels are known to the persons skilled in the art, e. g. a reference level can be determined from Receiver-Operator-Curves (ROC) according to clinical studies.

Furthermore, it will be appreciated that the chosen reference level will also depend on the method of measurement, e. g. depending on a particular activator and the time after which the level of the marker of platelet function is measured.

In a context of this discussion, it is evident, that a reference-level may not only be a single value, but it may also include a range of values.

More particularly, reference levels can also be derived from levels as determined e.g. in the studies according to the examples attached to this specification.

Guidance regarding particular reference levels may also be determined from the examples attached to this specification.

For example in the context of the determining whether Cyclooxygenase-1 inhibitors (Aspirin) or thromboxane A₂ (TXA2) receptor blockers (e. g., terbogrel) are effective in a patient, it can be assumed that a level of 0.5 - 0.6 ng/ml of sCD40L as measured after 6 minutes of in vitro activation may be able to distinguish between patients effective in reducing the capability of platelet aggregation or not. If, in this case, a patient shows a level of higher than 0.6 ng/ml of sCD40L after treatment with asperin or terbogrial it may indicate that such agent is not effective in this patent.

As another example, it can be seen that in a test for global functional platelet capacity (GFPC) or in a test for platelet stimulation capability (PSC) the level of sCD40L as measured according to the invention is increased in patients with acute coronary syndrome (unstable angina, non-ST-segment elevation myocardial infarction, or ST-segment elevation myocardial infarction).

In another example, the level of soluble GP-V as measured according to the invention is decreased at least 2 to 4 times in a subject receiving Aspirin (acetylsalicylic acid), indicating that such subject responds well to Aspirin treatment. Thus, a reference value can also be obtained by comparing values before and after treatment in a single subject.

If larger samples of subjects are analyzed, suitable reference values can be obtained. For example, a level below or above the average level plus or minus two standard deviations as determined from preferably more than 10, 20, 50, 100, 500 or 1000 comparable subjects may indicate that such subject is suffering from the relevant disorder.

The methods according to the invention may be carried out by any means deemed appropriate by the person skilled in the art. Therefore the present invention also relates to such devices and the use of such devices in a method according to the invention. Particularly considered are integrated devices able to carry out more than one step of the methods according to the invention. For example, a device for analyzing a blood sample is described in WO 99/14595 (Accumetrics Inc.) which is incorporated herein in its entirety by reference. Such device can receive a sample, e.g. a blood sample, from a sample container (e.g. a blood collection tube) and can move the sample through different chambers to carry out processing of the sample. The device has means for controlling the precise amount of the sample into the further chambers. Such device can be adapted to carry out at least the steps of (1) obtaining a sample from a blood sampling device or blood collection tube, (2) contacting any agent according to the present invention with the sample (e.g. a platelet activator or a protease), (3) (optionally) stopping the reaction of step (2), and (4) (optionally) measuring the level of a marker of platelet function. For step (4), the device can be adapted to comprise a means for measuring the level of the marker, such as known to the person skilled in the art. Examples for such means are also disclosed in this specification in the context of measuring the level of a peptide, polypeptide, or protein. The device may also be suitable or designed for taking a blood sample from a subject, e.g. it may comprise a means for directly transfering a blood sample from a patient into a reservoir instead of obtaining the sample from a blood sampling device or blood collection tube. In this case, the device may be considered to be a blood sampling device as described further below.

The invention also relates to a blood sampling device comprising (a) optionally an anticoagulant, (b) a platelet activator, (c) optionally an inhibitor platelet activation, and (d) optionally an inhibitor of fibrinolysis.

The term "blood sampling device" is known to the person skilled in the art. Preferably such device is a device suitable for taking a blood sample from a subject. Preferably, the blood sampling device is designed for taking a blood sample from a subject. Preferably, the blood sampling device comprises a reservoir portion into which a blood sample can be transferred. Preferably, the device allows to directly transfer or is designed to directly transfer a blood sample from a subject into the reservoir, e.g. by means of a needle and optionally a connection tube. Preferably, the blood sampling device comprises a container with an intake portion for the intake of a blood sample and a reservoir portion for receiving the sample. Preferably, the device allows contacting of the platelets in the blood sample with a platelet activator immediately on collection of the blood sample. Preferably, the device also allows contacting of the blood sample with an anticoagulant and/or an inhibitor of fibrinolysis (if any of such is comprised in the device) immediately on collection of the blood sample. The device may have a separating member (e.g. a mechanical separating element or a gel) for separating blood components.

The device may also allow to determine which amount of blood has been added to the device (e.g. by a being a transparent blood collection tube, preferably with an indicator line, which allows to visually determine the amount of sample added.

Preferably, the blood sampling device comprises means for indicating the amount of blood to be added, e.g. an indicator line or a label designating the amount to be added. Preferably, the device is transparent to allow visual control of the amount of blood added. However, the device may also comprise any other means deemed appropriate to match the amount of blood sample added with the amount of the agent (e.g. platelet activator, protease, or coagulation inhibitor) to be contacted with the sample.

The blood sampling device may also be color-coded or it may comprise a label indicating the contents and/or the purpose for which the tube is designed. Such purpose may be e.g. diagnosis of platelet function and/or dysfunction, or diagnosis of cardiovascular disorders or any other diagnostic purpose mentioned in this specification. Such information may also be provided in a package leaflet accompanying the device, e.g. in a kit or package comprising the device or a plurality of the devices.

The blood sampling device may be an evacuated system or a partially evacuated system. Evacuated or partially evacuated blood sampling devices, particularly blood collection tubes, are commercially available (e.g. from BD (Becton, Dickinson and Company)). The blood sampling device may also allow drawing a blood sample by aspiration. Such devices, particularly blood collection tubes, are commercially available (e.g. from Sarstedt AG, Germany). In a preferred embodiment such device comprises a plunger movable inside a tube capable of creating the suction (similar to a syringe). Preferably, it is possible to break off the plunger rod after aspiration of the sample. Such evacuated or partially evacuated systems or systems allowing to draw a sample by aspiration are particularly suited for directly transferring a blood sample or a blood platelet sample from a subject into the reservoir.

The blood sampling device may be manufactured from any kind of material deemed suitable. E.g. plastic or glass may be used. Some preferred materials used to manufacture the device (particularly a blood collection tube) include polypropylene, polyethylene, polyethyleneterephthalate, polystyrene, polycarbonate and cellulosics. More expensive plastics such as polytetrafluoroethylene and other fluorinated polymers may also be used. In addition to the materials mentioned above, examples of other suitable materials for the collection devices used in the present invention include polyolefins, polyamides, polyesters, silicones, polyurethanes, epoxies, acrylics, polyacrylates, polysulfones, polymethacrylates, PEEK, polyimide and fluoropolymers such as PTFE Teflon^{™}, FEP Teflon^{™}, Tefzel^{™}, poly (vinylidene fluoride), PVDF and perfluoroalkoxy resins. Glass products including silica glass are also used to manufacture the collection devices. One exemplary glass product is PYREX^{™} (available from Coming Glass, Coming, New York). Ceramic collection devices can be used according to embodiments of the invention. Cellulosic products such as paper and reinforced paper containers can also be used to form collection devices according to the invention.

The agent to be contacted with the sample (e.g. platelet activator, protease, anticoagulant, fibrinolysis inhibitor, and/or platelet inhibitor) may be in any suitable form including, but not limited to, a solution, suspension or other liquid, a pellet, a tablet, a capsule, a spraydried material, a freeze-dried material, a powder, a particle, a gel, crystals or a lyophilized material. Because the half-life of some platelet activators, anticoagulants, fibrinolysis inhibitors, and platelet inhibitors may be short, the relevant component is preferably introduced into the blood sampling device in such a form so as to optimize the shelf life. Lyophilization appears to be useful in that it provides good stability and also allows subsequent sterilization, both of which are key from a standpoint of automation and standardization.

Said agent (e.g. platelet activator, protease, anticoagulant, fibrinolysis inhibitor, and/or platelet inhibitor) may be located anywhere in the device, e.g. on a surface which can come into contact with the blood sample. Said agent may also be located in a connection (e.g. connection tube) between the patient and the device. Said agent may also be located on stoppers and seals for closing such devices or on mechanical, or other, inserts placed within such devices. Preferably, the agent is located anywhere along at least one interior wall of the collection device or anywhere within the reservoir portion. In addition, some agents may exhibit light sensitivity. Thus, it may be desirable to protect the agent from light. For such agents, use of an opaque tube, e. g. an amber-colored tube, would be advantageous. Alternatively, placing the agent into a capsule that protects it from light exposure, e. g., in powdered form, and then placing the capsule into the tube would also address this issue. Capsulating the agent may also prevent other undesirable interactions between the agent and other elements in the container. Capsule materials that dissolve upon sample collection are well known in the art.

The platelet activator, anticoagulant, fibrinolysis inhibitor, and/or platelet inhibitor may be applied to the blood sampling device by any number of methods. For example, the agent may be spray dried, loosely dispensed or lyophilized over the surface of the interior wall of the blood sampling device. Alternatively, the agent, such as when in gel or liquid form, for example, may be positioned in the reservoir portion of the blood sampling device. Additional methods for providing the blood sampling device with the platelet activator, anticoagulant, fibrinolysis inhibitor, and/or platelet inhibitor are also possible. Typically, to dispose the desired amount of agent into a container, one reconstitutes a solid form of the agent and then dispenses the appropriate amount of liquid into the container. The liquid may be spray dried, disposed into the bottom of the container or subsequently lyophilized.

Preferably, the amount of activator contained in the device for activating the platelets is capable of reliably activating the platelets in the sample, as defined elsewhere in this specification. Preferably, the amount of platelet activator is sufficient to result in shedding of sCD40L from the CD40L present on the surface of the platelets in the sample, more particularly in shedding of at least 30%, at least 50%, at least 70%, at least 80%, at least 90%, or at least 95% of the total amount of CD40L present on the platelet surface within less than 10, 8, 7, 5, 3 or 2 minutes. Preferably the amount of activator is sufficient to result in shape changes typical of platelet activation within less than 10, 8, 7, 5, 3 or 2 minutes.

Preferably, the amount of activator is sufficient to have at least the platelet activating ability of 0.05 µg/ml, more preferably 0.1µg/ml, 0.2 µg/ml, 0.5 µg/ml or 1 µg/ml of collagen in a blood sample. More preferably, the amount of activator corresponds to the platelet activating ability of 0.05 to 50 µg/ml, 0.2 to 30 µg/ml, 0.5 to 10 µg/ml, 0.8 to 5 µg/ml, or 1 µg/ml of collagen in a blood sample (preferably in a freshly obtained whole blood sample).

Alternatively or additionally, the amount of activator is preferably sufficient to have at least the platelet activating ability of 0.01 µM, more preferably 0.1 µM, 0.2 µM, 0.5 µM, 1 µM, 2 µM, 3 µM or 5 µM of ADP in a blood sample. More preferably, the amount of activator corresponds to the platelet activating ability of 0.01 µM to 100 µM, 0.1 to 50 µM, 0.2 to 30 µM, 0.5 to 20 µM, 2 to 15 µM, 3 to 10 µM or 5 µM of ADP in a blood sample (preferably in a freshly obtained whole blood sample).

Alternatively or additionally, the amount of activator is preferably sufficient to have at least the platelet activating ability of 0.01 U/ml, more preferably 0.05 U/ml, 0.1 U/ml, 0.2 U/ml, 0.5 U/ml or 1 µg/ml of collagen in a blood sample. More preferably, the amount of activator corresponds to the platelet activating ability of 0.01 to 10 U/ml, 0.05 to 10 U/ml, 0.1 to 10 U/ml, 0.2 to 8 U/ml, 0.5 to 5 U/ml,or 1 U/ml of thrombin in a blood sample (preferably in a freshly obtained whole blood sample).

A typical blood sampling device may include a container defining an internal chamber. The container may be a hollow tube having a side wall, a closed bottom end, and an open top end. Optionally, a separating member may be provided within the container chamber. Such a separating member may serve to assist in separating components of the sample, for example, by centrifugation. The container is dimensioned for collecting a suitable volume of blood. A closure means for covering the open end to close the container is preferably and may be necessary where a sterile product is demanded. For conventional tubes, a screw cap is normally sufficient. For evacuated collection tubes, a tight-fitting, elastomeric plug is generally employed to contain the vacuum during the required storage periods. Preferably, the closure means forms a seal capable of effectively closing the container and retaining a biological sample in the internal chamber. The closure may be one of a variety of forms including, but not limited to, rubber closures, HEMOGUARD closures, metallic seals, metal-banded rubber seals and seals of different polymers and designs. A protective shield may overlie the closure means. The container may also contain the platelet activator, and optionally an anticoagulant, fibrinolysis inhibitor, and/or platelet inhibitor. The container can be made of glass, plastic or other suitable materials. Preferably, the container is transparent. Non-limiting examples of suitable transparent thermoplastic materials for the container are polycarbonates, polyethylene, polypropylene and polyethyleneterephthalate. Plastic materials can be oxygen impermeable materials or may contain an oxygen impermeable or semi-permeable layer. Alternatively, the container can be made of a water and air permeable plastic material. The platelet activator, and optionally an anticoagulant, fibrinolysis inhibitor, and/or platelet inhibitor may be provided to the container using any appropriate means. In one aspect, the relevant agent (platelet activator, optionally anticoagulant, optionally fibrinolysis inhibitor, and/or optionally platelet inhibitor) is in a liquid solution and is placed into the container. Subsequently, the solution may be lyophilized by methods that are known in the art such as, for example, freeze drying. For example, by freezing the solution and then slowly warming after freezing, while simultaneously applying a vacuum, a freeze-dried powder remains in the collection tube. An additive such as an excipient, for example, PVP or trehalose, may also be added to the agent solution prior to freeze drying so that the resulting agent is pelletized in the container. Vacuum drying may also be used after adding the stabilizing solution. In another aspect, the stabilizing agent is formed into a liquid or solid aerosol and sprayed onto one or more surfaces of the interior of the container.

If the tube is an evacuated or partially evacuated system, the pressure in the internal chamber may be selected to draw a predetermined volume of biological sample into the chamber. Preferably, the closure means is made of a resilient material that is capable of maintaining the internal pressure differential between atmospheric pressure and a pressure less than atmospheric. The closure means is such that it can be pierced by a needle or other cannula to introduce a sample into the container as known in the art. Preferably, the closure means is resealable. Suitable materials for the closure means include, for example, silicone rubber, natural rubber, styrene butadiene rubber, ethylene-propylene copolymers and polychloroprene.

Suitable examples of a container include single-wall and multi-layer tubes. A more specific example of a suitable container is disclosed in U. S. Patent No. 5,860, 937 to Cohen, which is hereby incorporated by reference in its entirety.

A blood sampling device according to the invention may be manufactured comprising the steps of (a) providing container suitable for use in a blood sampling device, said container preferably having a reservoir portion for receiving the sample, (b) adding a platelet activator to the container, preferably to the reservoir portion, (c) optionally adding an anticoagulant and/or a fibrinolysis inhibitor and/or a platelet inhibitor to the container, preferably to the reservoir portion of the container, (d) optionally lyophilizing any of the agents added according to steps (b) or (c), (e) assembling the device, (f) optionally evacuating the container. The agent according to step (b) or (c) may be dispensed into the container e.g. in solution form or as a pellet, tablet, particle, crystal or capsule. After adding the agents to the container, a separating member may be added to the container, if desired. An example of a suitable lyophilization/evacuation process is as follows: the container is frozen at a temperature of about -40 °C at a pressure of about 760 mm Hg for about 6 to 8 hours; the container is dried as the temperature is ramped from -40 °C to about 25 °C, at a pressure of about 0.05 mm Hg, for about 8 to 10 hours; and the container is then evacuated at a temperature of about 25 °C and a pressure of about 120 mm Hg for about 0.1 hours. Preferably, the sterilization technique is with cobalt 60 radiation.

As noted, the container may also contain a gel, mechanical or other separating member (e.g., filter paper or the like). In such cases, the relevant agent may be spray dried and/or lyophilized on an exterior surface of the separation media. The container may also allow blood plasma preparation. Such a device comprises, in addition to the platelet activator and other relevant agent(s), an element for separating plasma from whole blood. The element for separating plasma from whole blood may be a separating member such as a gel formulation or a mechanical media. The gel is desirably a thixotropic polymeric gel formulation. The gel may be a homopolymer or a copolymer and may include silicone-based gels such as, for example, polysiloxanes, or organic hydrocarbon-based gels such as, for example, polyacrylics, polyesters, polyolefins, oxidized cis polybutadienes, polybutenes, blends of epoxidized soybean oil and chlorinated hydrocarbons, copolymers of diacids and propandiols, hydrogenated cyclopentadienes and copolymers of alphaolefins with dialkylmaleates. The gel desirably isolates the plasma from the cells of the blood sample in the tube by serving as a density separation medium. In this way, after centrifugation the plasma is separated from the blood. In the case of a gel separating material, it may be desirable to provide physical/chemical separation between the relevant agent and the gel,e. g., use of a capsule as discussed above. For example, if portions of the agent are incorporated into or react with the gel, the effectiveness of the agent may be reduced. For the same reasons, where a mechanical separating element is used, the element is desirably substantially inert to the stabilizing agent, and this reflects an advantage of such a separator. Providing a separating element in plasma tubes, versus centrifuging without a separating element, may be advantageous as it allows to maintain the sample without excessive further leakage of a marker of platelet function into the supernatant.

Blood sampling devices are commercially available. Particularly, also blood sampling devices comprising an inhibitor of coagulation are commercially available. An example of such a blood sampling device comprising an inhibitor of coagulation is disclosed in U. S.

Patent No. 5,667, 963 to Smith et al. and in WO 03/097237 A2, which are herein incorporated in their entirety by reference.

Examples for a blood sampling device according to the invention include closed system blood collection devices (such as collection bags), syringes (especially pre-filled syringes), and blood collection tubes.

An example for a blood sampling device is a blood collection tube. The term "blood collection tube" is known to the person skilled in the art. Typically, a blood collection tube comprises an intake portion for the intake of a blood sample and a reservoir portion for receiving the sample. Preferably, the intake portion comprises a connecting means for connecting the blood collection tube to a needle or connection tube, more particularly by means of a specific adaptor, e.g. a Luer-Lok or Multifly^{™} (Sarstedt AG, Germany). The connection may be sealable, e.g. by means of a rubber insert capable of sealing the intake portion after it has been perforated with a needle. Thus, a needle or connection tube can be inserted to allow for introducing a sample but sealing the tube off after a sample has been added. The intake portion may comprise a screw cap or it may be comprised in a screw cap which can be screwed onto the reservoir portion of the collection tube.

Typically, a blood collection tube comprises a sampling reservoir for 0.1 to 25 ml, particularly 0.5 ml to 20 ml, more particularly 0.8 to 15 ml, more particularly 1 to 10 ml blood sample. Typically, a blood collection tube has a size corresponding to (a) a length of 2 to 20 cm, particularly 4 to 18 cm, more particularly 5 to 15 cm and (b) an inner diameter of 5 to 20 mm, particularly 6 to 18 mm, more particularly 7 to 16 mm.

It is apparent that the blood collection tube may be adapted according to any features already mentioned in the context of blood sampling devices, particularly concerning color codes, closures, means for indicating the amount of sample to be added, materials, agents to be added, forms or means of adding said agents, means for aspirating a sample, etc.).

Neutral tubes without filling are available e.g. as Sarstedt Monovette^{™} catalogue no. 01.1728.001, 04.1926.001, and 05.1727.001 (Sarstedt AG, Germany).

Tubes with coagulation inhibitor are available e.g. as Sarstedt Monovette^{™} catalogue no. 01.1604, 03.1628, 04.1936, and 04.1906 (all containing lithium-heparin) and 04.1909 (containing CTAD).

Other commercially available blood collection tubes include tubes available als VACUTAINER^{™} from BD (Becton, Dickinson and Company).

In one embodiment, an inhibitor of platelet activation is also provided in the blood sampling device or it may be added to the sample in the device after activation of the platelets. For example, the inhibitor may be added manually to the device, e.g. by means of a syringe. Thus, it is possible to stop the activation of the platelets, particularly after a predetermined time-period.

In a more particular embodiment, the device comprises the inhibitor of platelet activation in a mode that enables a release of the inhibitor into a blood-platelet containing sample after the addition of the blood platelet-containing sample and after bringing the marker of platelet function or variant thereof into solution. More particularly, the inhibitor of platelet activation may be provided in a mode releasable or providing for release after a particular time-period. For example, the inhibitor may be separated from the sample by a separating means allowing for release after a certain time of contact with the sample, e.g. by manually removing or breaking said separating means. Said separating means may also dissolve upon a predetermined time of contact with the sample (e.g. an aqueous solution). Preferably, the predetermined time corresponds to any of the suitable time-periods for activation as mentioned in this specification. Said separating means may be e.g. solid material, a gel, or a membrane separating or encapsulating the inhibitor.

The inhibitor may also be provided as a more slowly acting agent as compared to the platelet activator. Thus, the inhibitor will counteract the activator only after a predetermined time. An example for such inhibitor is tirofiban, see Example 8).

In another embodiment, a proteolysis inhibitor is also provided in the blood sampling device or it may be added to the sample in the device after solubilizing the marker of platelet function by means of proteolysis. For example, the inhibitor may be added manually to the device, e.g. by means of a syringe. Thus, it is possible to stop the proteolysis, particularly after a predetermined time-period.

In a more particular embodiment, the device comprises the proteolysis inhibitor in a mode that enables a release of the inhibitor into a blood-platelet containing sample after the addition of the blood platelet-containing sample and after bringing the marker of platelet function or variant thereof into solution. More particularly, the proteolysis inhibitor may be provided in a mode releasable or providing for release after a particular time-period. For example, the inhibitor may be separated from the sample by a separating means allowing for release after a certain time of contact with the sample, e.g. by manually removing or breaking said separating means. Said separating means may also dissolve upon a predetermined time of contact with the sample (e.g. an aqueous solution). Preferably, the predetermined time corresponds to a suitable time-period for solubilizing the marker of platelet function as mentioned in this specification. Said separating means may be e.g. solid material, a gel, or a membrane separating or encapsulating the inhibitor.

The invention also relates to the use use of a kit comprising (a) a blood sampling device and/or any other device as laid out in this specification, (b) a platelet activator, (c) optionally, an anticoagulant, (d) optionally an inhibitor of platelet activation, and (e) optionally an inhibitor of fibrinolysis.

The kit may also comprise a means or agent for measuring a marker of platelet function as defined elsewhere in this specification. Such means or agent may be any suitable means or agent known to the person skilled in the art. Examples for such means or agents as well as methods for their use have been given in this specification. For example, a suitable agent may be any kind of ligand or antibody capapble of specifically binding to a marker of platelet function, e.g. to sCD40L or P-Selectin. The kit may also comprise any other components deemed appropriate in the context of measuring the level(s) of the respective biomarkers, such as suitable buffers, filters, etc.

Optionally, the blood sampling device, blood collection tube, or kit may additionally comprise a user's manual for interpreting the results of any measurement(s) with respect to diagnosing platelet function, platelet dysfunction, a dysfunction in which platelets are stimulated, or any other diagnoses or uses as laid out in this specification. Particularly, such manual may include information about which measured level corresponds to which grade of platelet function or to what kind of diagnosis (e.g dysfunction present or not present). Additionally, such user's manual may provide instructions about correctly using the components of the kit for obtaining a blood platelet sample, and/or for activating the platelets, and/or for measuring the level(s) of the respective marker of platelet function. Particularly, the user's manual may provide instructions about the time required until platelet activation is stopped.

The invention also relates to the use of said blood sampling device, blood collection tube, or kit for diagnosing platelet function, platelet dysfunction, or a dysfunction in which platelets are stimulated, or any other diagnoses or uses as laid out in this specification.

The invention also relates to any compositions mentioned in this specification or the examples as well as to any samples comprising such compositions. The invention also relates to any preferred embodiments of such compositions or samples such as paricular concentrations or combinations as mentioned anywhere in the specification or examples.

### Figure legends:

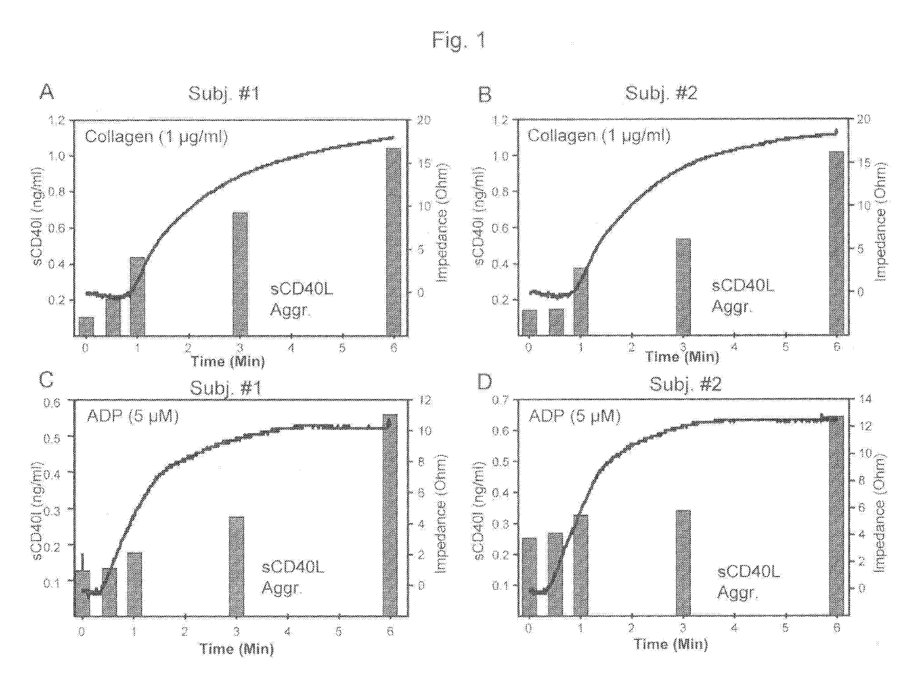

Fig. 1: sCD401 concentrations were determined in diluted whole blood (1:1 with saline) at several time points after aggregation was started by addition of agonists collagen (1 µg/ml) or ADP (5 µM). The course of aggregation was followed using impedance aggregometry (Ohm). Ag., Agonist; Subj. #1, subject No. 1; subj. #2, subject #2; Aggr., Aggregation. Time, time after additions of agonist. The bars indicate the level of sCD40L. The black curve shows the aggregation curve as determined by inpedance aggregometry.

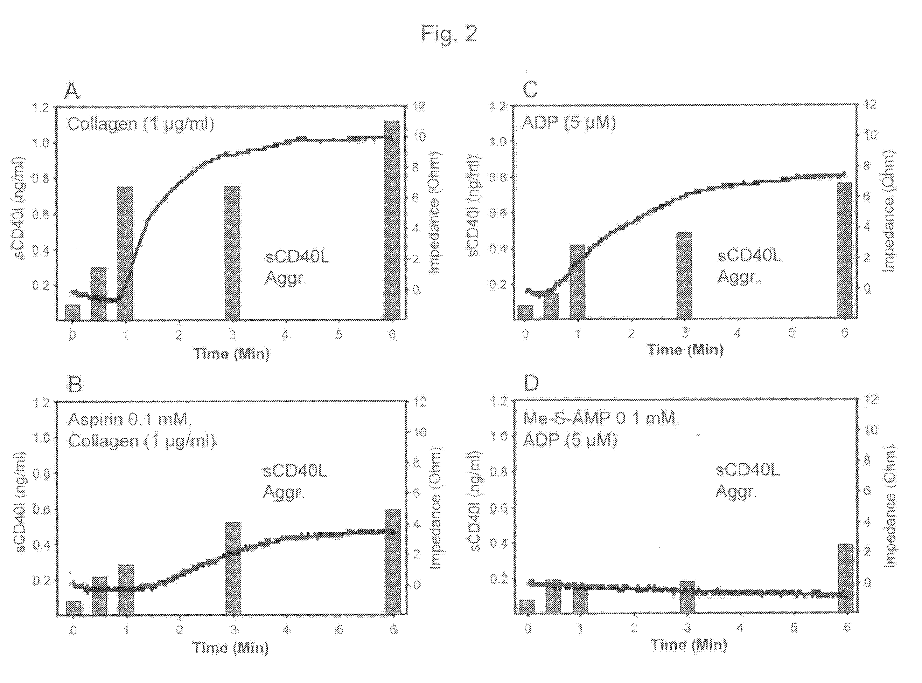

Fig. 2: sCD40L concentrations were determined in diluted whole blood (1:1 with saline) at several time points after aggregation was started by addition of agonists collagen (1 µg/ml) or ADP (5 µM). Panels B and D show the results after preincubation with two known inhibitors of platelet activation (aspirin and methyl-S-adenosine mono-phosphate (Me-S-AMP)). The course of aggregation was followed using impedance aggregometry (Ohm). Ag., Agonist; Aggr., Aggregation. Time, time after additions of agonist. The bars indicate the level of sCD40L. The black curve shows the aggregation curve as determined by inpedance aggregometry.

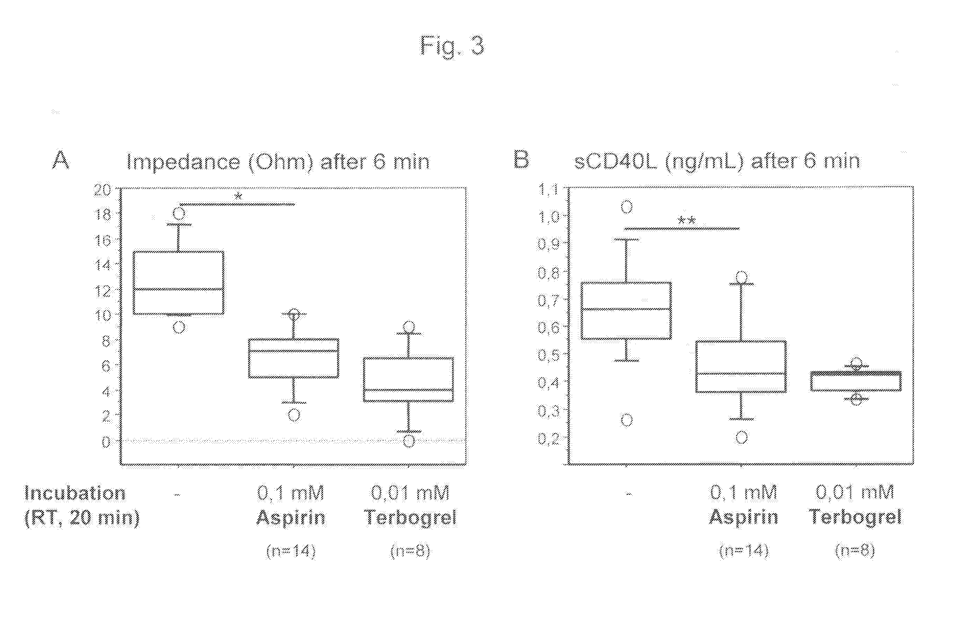

Fig. 3: Influence of Cyclooxygenase-1 (COX1) Inhibitors (Aspirin) and Thromboxane A2 (TXA2)-Receptor Blocker (Terbogrel) on sCD40L Release and Platelet Aggregation. Blood samples were obtained from healthy blood donors (no intake of platelet inhibitors during the preceding week). All samples were analysed directly (n=14) and after incubation (20 min, room temperature) with either Aspirin (n=14) or Terbogrel (n=8), a combined COX-1 and TXA2-receptor blocker. After dilution (1:1) with NaCl, aggregation was started by addition of collagen (1 µg/ml) and was monitored by impedance aggregometry. After 6 min, aggregation was stopped by centrifugation and sCD40L was analysed in the plasma supernatants. n-= number of subjects investigated. *) p = 0,001 (Wilcoxon-Signed Rank) **) p = 0,005

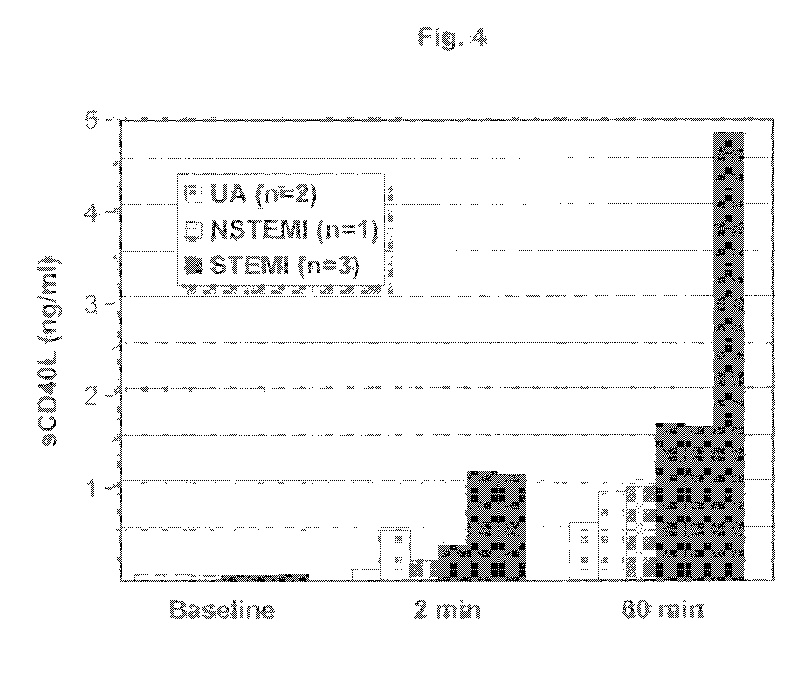

Fig. 4: sCD40L released by TRAP (2 µM) after 2 min and 60 min (GFPC). Citrate-anticoagulated blood samples from patients (n=6) with acute coronary syndrome (ACS): unstable angina (UA), non-ST-segment elevation myocardial infarction (NSTEMI), ST-segment elevation myocardial infarction (STEMI). All patients received Aspirin (500 mg iv) and heparin (5000 U bolus iv) at least 30 min before sampling. Baseline: Measurement after obtaining the sample without activation in vitro.

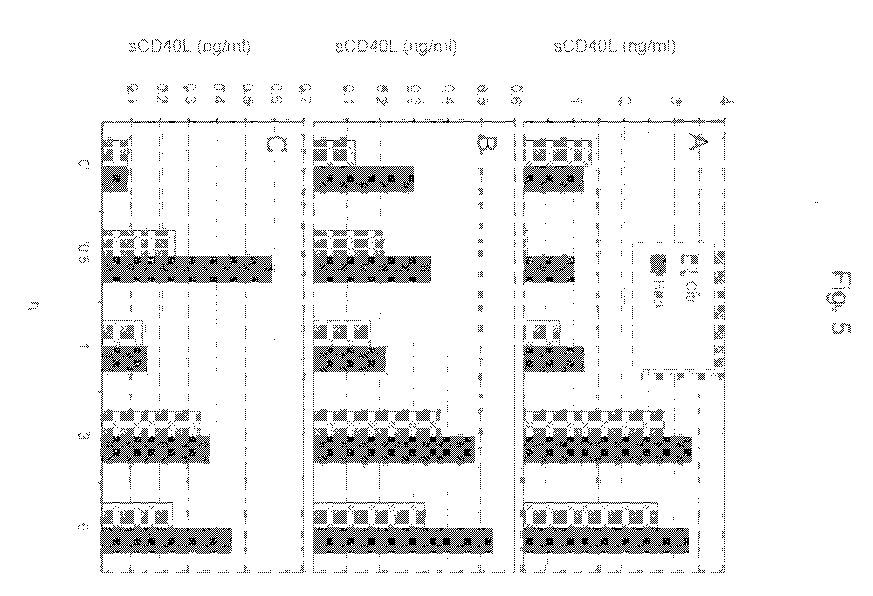

Fig. 5: The figure shows the effect of incubation at room temperature prior to standardized platelet activation for 10 min using 2 µM TRAP (A), 10 µM ADP (B), and 1 µg/ml collagen (C) as agonists. Citrate- and heparin-anticoagulated blood was obtained from a healthy volunteer. The release of sCD40L during activation is influenced most by incubation time and less by the choice of agonist or anti-coagulant. Therefore,it is preferable to start standardized platelet activation right at the moment of blood sampling. h, hours; Citr., citrate; Hep., heparin.

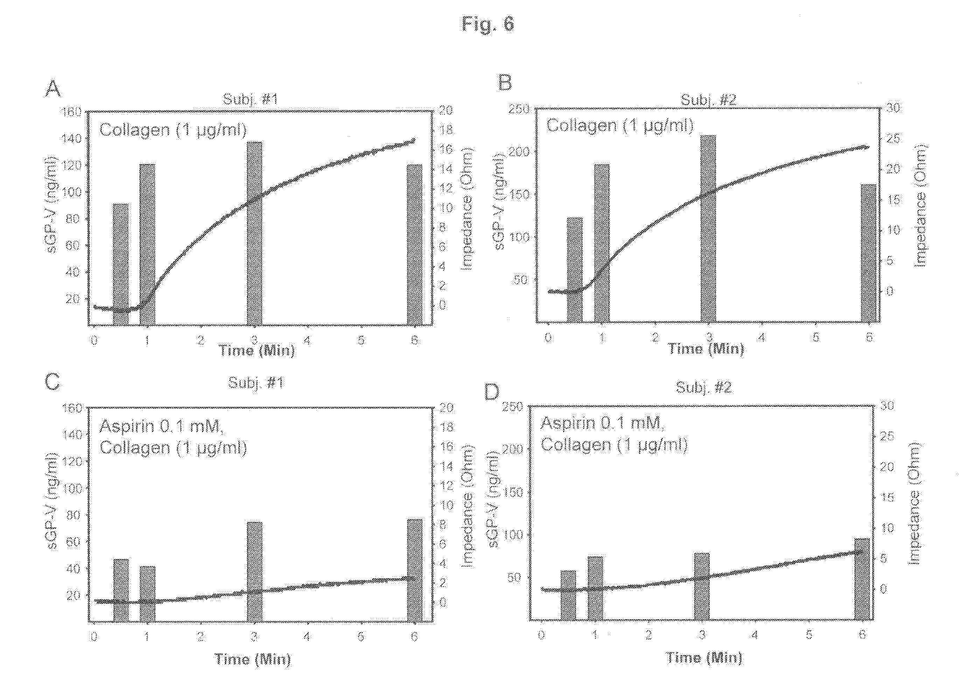

Fig. 6: Release of GP-V as soluble GP-V (sGP-V) after activation in vitro. Subj. #1, subject #1, subj. #2, subject #2.

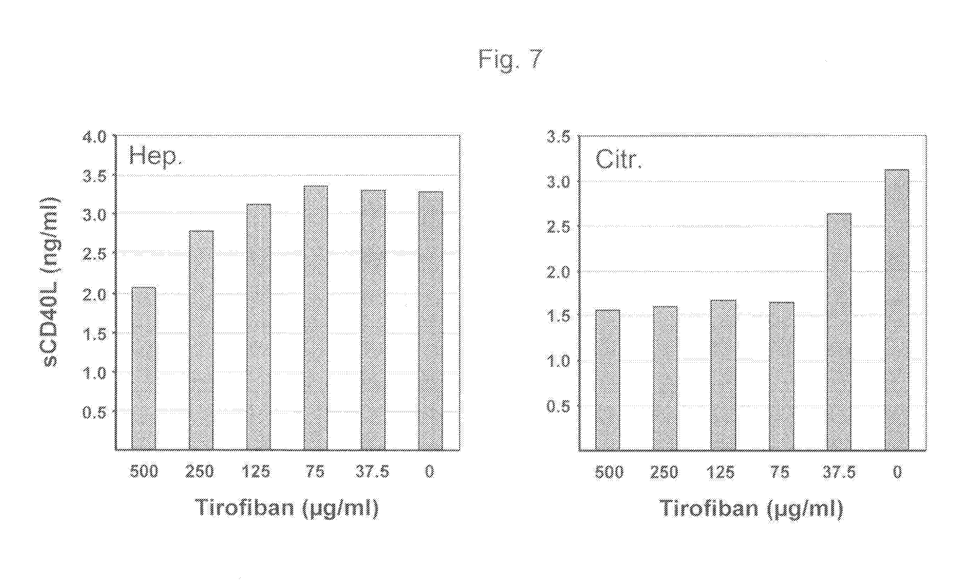

Fig. 7: sCD40L as determined in presence of heparin (Hep.) and citrate (citr.) using tirofiban as combined actiar and inhibitor.

### Examples:

**Example 1:** Citrate-anticoagulated blood was taken from 2 healthy subjects who did not take any antiplatelet medication during the preceding 10 days. Whole blood was diluted with 9g/l (1:1) and analysed by 4-channel impedance aggregometry (Chrono-Log 560CA and 590, Havertown, PA, U.S.A.) following the instructions of the manufacturer. Reagents for aggregometry were obtained from Chono-Log. Platelet aggregation was started by addition of collagen or adenosine di-phosphate (ADP) to obtain final concentrations of 1 µg/ml or 5 µM, respectively. In 3 channels, aggregation was stopped after 0.5, 1, and 3 min, respectively, by addition of the glycoprotein IIb/IIIa inhibitor tirofiban and immediate centrifugation (13.000 rpm for 20 min at room temperature). In the last channel, aggregation was followed as increase of impedance (Ohm). After 6 min, the aggregation was stopped in this channel as described above. Another diluted blood sample was stopped immediately (baseline, 0 min sample). After centrifugation, sCD40L was measured in the sample supernatants by use of an assay for the Elecsys 2010 automated analyser (Roche Diagnostics GmbH, Germany). The results are shown in Table 1.

**Table 1:**

| | **Subj. #1** | | | | **Subj. #2** | | | |
|---|---|---|---|---|---|---|---|---|
| **Ag.** | **Collagen (1µg/ml)** | | **ADP (5µM)** | | **Collagen (1µg/ml)** | | **ADP (5µM)** | |
| **min** | **sCD40L (ng/ml)** | **Aggr. (Ohm)** | **sCD40L (ng/ml)** | **Aggr. (Ohm)** | **sCD40L (ng/ml)** | **Aggr. (Ohm)** | **sCD40L (ng/ml)** | **Aggr. (Ohm)** |
| 0 | 0.106 | 0 | 0.128 | 0 | 0.144 | 0 | 0.254 | 0 |
| 0.5 | 0.206 | 0 | 0.136 | 0.8 | 0.152 | 0 | 0.274 | 1.0 |
| 1.0 | 0.438 | 1.4 | 0.178 | 4.5 | 0.382 | 1.4 | 0.330 | 5.9 |
| 3.0 | 0.694 | 13.5 | 0.282 | 9.6 | 0.540 | 14.4 | 0.346 | 12.1 |
| 6.0 | 1.042 | 18.0 | 0.560 | 10.3 | 1.016 | 18.3 | 0.646 | 12.6 |

**Example 2:** Increasing sCD40L solubilisation during whole blood aggregation, see Fig. 1. Citrate-anticoagulated blood was taken from a healthy subject (Fig 1) who did not take any antiplatelet medication during the preceding 10 days. Whole blood was diluted with 9g/l NaCl₂ (1:1) and analysed by 4-channel impedance aggregometry (Chrono-Log 560CA and 590, Havertown, PA, U.S.A.) following the instructions of the manufacturer. Reagents for aggregometry were obtained from Chono-Log. Platelet aggregation was started by addition of collagen (graphs A, B) or adenosine di-phosphate (ADP, graphs C, D) to obtain final concentrations of 1 µg/ml or 5 µM, respectively. In 3 channels, aggregation was stopped after 0.5, 1, and 3 min, respectively, by addition of the glycoprotein IIb/IIIa inhibitor tirofiban and immediate centrifugation (13.000 rpm for 20 min at room temperature). In the last channel, aggregation was followed as increase of impedance (Ohm) illustrated as a line in each graph. After 6 min, the aggregation was stopped in this channel as described above. Another diluted blood sample was stopped immediately (baseline, 0 min sample). After centrifugation, sCD40L was measured in the sample supernatants by use of an assay for the Elecsys 2010 automated analyser (Roche Diagnostics GmbH, Germany). sCD40L concentrations are illustrated in each graph by bars placed at the time points at which the aggregation was stopped.

**Example 3:** Increasing sCD40L solubilisation during whole blood aggregation in response to platelet agonists collagen (Fig. 2, graphs A, C) and adenosine phosphate (ADP), Fig. 2, graphs B, D. Aggregation and sCD40L solubilisation were markedly reduced in a concordant fashion following pre-incubation (20 min at room temperature) with 2 known inhibitors of platelet activation and aggregation, i. e. 0.1 mM, Fig 2, aspirin (graph C) and 0.1 mM Me-S-AMP (methyl-S-adenosine mono-phosphate (Sigma-Aldrich Inc.), Fig. 2, graph D).
Citrate-anticoagulated blood was taken from a healthy subject who did not take any antiplatelet medication during the preceding 10 days. Whole blood was diluted with 9g/l NaCl (1:1) and analysed by 4-channel impedance aggregometry (Chrono-Log 560CA and 590, Havertown, PA, U.S.A.) following the instructions of the manufacturer. Reagents for aggregometry were obtained from Chono-Log. Platelet aggregation was started by addition of collagen (Fig. 2, graphs A, C) or adenosine di-phosphate (ADP), (Fig. 2, graphs B, D) to obtain final concentrations of 1 µg/ml or 5 µM, respectively. In 3 channels, aggregation was stopped after 0.5, 1, and 3 min, respectively, by addition of the glycoprotein IIb/IIIa inhibitor tirofiban and immediate centrifugation (13.000 rpm for 20 min at room temperature). In the last channel, aggregation was followed as increase of impedance (Ohm) illustrated as a line in each graph. After 6 min, the aggregation was stopped in this channel as described above. Another diluted blood sample was was stopped immediately (baseline, 0 min sample). After centrifugation, sCD40L was measured in the sample supernatants by use of an assay for the Elecsys 2010 automated analyser (Roche Diagnostics GmbH, Germany). sCD40L concentrations are illustrated in each graph by bars placed at the time points at which the aggregation was stopped.

**Example 4:** Whole blood aggregation (Fig. 3, left graph) and sCD40L solubilisation during aggregation (Fig. 3, right graph) are significantly inhibited after incubation with 2 known inhibitors of platelet aggregation, i. e. the cylcooxygenase-1 inhibitor aspirin or the thromboxane-A2 receptor blocker terbogrel. Aggregation (6-min impedance) and sCD40L concentrations after 6-min aggregation are illustrated as box plots.
Citrate-anticoagulated blood was obtained from 14 healthy blood donors who did not take any antiplatelet medication during the preceding 10 days. Aliquots of the whole blood samples were incubated (20 min at room temperature) with 9g/l NaCl, aspirin (0.1 mM) or, in some samples, with terbogrel (0.01 mM). Aliquots were then diluted with 9g/l NaCl (1:1) and analysed by 4-channel impedance aggregometry (Chrono-Log 560CA and 590, Havertown, PA, U.S.A.) following the instructions of the manufacturer. Reagents for aggregometry were obtained from Chono-Log. Platelet aggregation was started by addition of collagen to obtain final concentrations of 1 µg/ml. Then, aggregation was monitored as increase of impedance (Ohm). After 6 min, the aggregation was stopped by addition of the glycoprotein IIb/IIIa inhibitor tirofiban and immediate centrifugation (13.000 rpm for 20 min at room temperature). After centrifugation, sCD40L was measured in the sample supernatants by use of an assay for the Elecsys 2010 automated analyser (Roche Diagnostics GmbH, Germany). Statistical analysis was done by use of the Wilcoxon-signed rank test.

**Example 5:** sCD40L concentration in blood samples from patients (n=6) with acute coronary syndrome (ACS) after platelet activation induced by thrombin-receptor activating peptide (TRAP, Sigma-Aldrich Inc.). Citrate-anticoagulated blood samples were obtained from patients presenting with unstable angina (UA), non-ST-segment elevation myocardial infarction (NSTEMI), and ST-segment elevation myocardial infarction (STEMI). All patients received aspirin (500 mg iv) and heparin (5000 U bolus iv) at least 30 min before sampling. Sample aliquots were incubated with 2 µM TRAP for 2 and 60 min at room temperature, respectively, followed by immediate centrifugation (13.000 rpm for 20 min at room temperature). Baseline values were obtained by centrifugation of blood samples immediately after blood sampling. After centrifugation, sCD40L was measured in the sample plasma by use of an assay for the Elecsys 2010 automated analyser (Roche Diagnostics GmbH, Germany). The results are shown in Fig. 4.

**Example 6:** Influence of delayed platelet activation on the sCD40L solubilisation achieved by use of thrombin-receptor activating peptide (TRAP), (Fig. 5, A), 10 µM ADP (Fig. 5, B), or 1 µg/ml collagen (Fig. 5, C). The solubilisation of sCD40L by platelet activation was influenced most by delayed sample processing and less by the choice of agonist or anticoagulant.
Citrate- and heparin-anticoagulated blood was obtained from a healthy subject who did not take any antiplatelet medication during the preceding 10 days. Samples were immediately processed further (0 hours) or left unattended at room temperature for 0.5, 1, 3, or 6 hours. Then, samples were incubated with 2 µM TRAP for 10 min followed by centrifugation (13.000 rpm for 20 min at room temperature). After centrifugation, sCD40L was measured in the sample supernatants by use of an assay for the Elecsys 2010 automated analyser (Roche Diagnostics GmbH, Germany). The results are shown in Fig. 5.

**Example 7:** Illustration of increasing solubilisation of glycoprotein V (sGpV) during whole blood aggregation in response to platelet agonist collagen, see Fig. 6. Aggregation and sCD40L solubilisation were markedly reduced in a concordant fashion following pre-incubation (20 min at room temperature) with aspirin (0.1 mM, graph C, D), a known inhibitor of platelet aggregation.
Citrate-anticoagulated blood was taken from 2 healthy subjects (A, C, and B, D, respectively) who did not take any antiplatelet medication during the preceding 10 days. Whole blood was diluted with 9 g/l NaCl (1:1) and analysed by 4-channel impedance aggregometry (Chrono-Log 560CA and 590, Havertown, PA, U.S.A.) following the instructions of the manufacturer. Reagents for aggregometry were obtained from Chono-Log. Platelet aggregation was started by addition of collagen to obtain a final concentration of 1 µg/ml. In 3 channels, aggregation was stopped after 0.5, 1, and 3 min, respectively, by addition of the glycoprotein IIb/IIIa inhibitor tirofiban and immediate centrifugation (13.000 rpm for 20 min at room temperature). In the last channel, aggregation was followed as increase of impedance (Ohm) illustrated as a line in each graph of Fig. 6. After 6 min, the aggregation was stopped in this channel as described above. After centrifugation, sCD40L was measured in the sample supernatants by use of an assay for the Elecsys 2010 automated analyser (Roche Diagnostics GmbH, Germany). sCD40L concentrations are illustrated in each graph of Fig. 6 by bars placed at the time points at which the aggregation was stopped.

**Example 8:** Concentration-dependent inhibition of sCD40L solubilisation by tirofiban, a Gp IIb/IIIa receptor blocker, see Fig. 7.
Citrate- (Fig. 7, right graph) and heparin-anticoagulated blood (Fig. 7, left graph) was obtained from a healthy subject who did not take any antiplatelet medication during the preceding 10 days. Sample aliquots were incubated (15 min, room temperature) with thrombin-receptor activating peptide (2 µM, TRAP) in the presence of decreasing concentrations of tirofiban (500, 250, 125, 75, 37.5, 0 µg/ml) followed by centrifugation (13.000 rpm for 20 min at room temperature). After centrifugation, sCD40L concentrations were measured in the sample supernatants by use of an assay for the Elecsys 2010 automated analyser (Roche Diagnostics GmbH, Germany).

## Claims

1. A method for diagnosing platelet function comprising the steps of
a) obtaining a blood platelet-containing sample from a subject, and
b) keeping the sample under conditions capable of inhibiting coagulation and/or fibrin formation,
c) bringing a marker of platelet function derived from the platelets, or a variant of such marker into solution,
wherein at least steps b) and c) are carried out in a blood sampling device and/or wherein additionally the level of the marker of platelet function in the solution is measured.

2. The method according to claim 1, wherein step (c) is carried out immediately after step a).

3. The method according to any of claims 1 to 2, wherein in step (c) the sample is contacted with a platelet activator or a protease.

4. The method according to any of claims 1 to 3, wherein in step (b) the sample is contacted with an anticoagulant.

5. The method according to any of claims 1 to 4, comprising the additional step of d) stopping the reaction, preferably after a pre-determined time-period.

6. The method according to any of claims 1 to 5, wherein the marker is differentially expressed correlating with different degrees of stimulation of platelets in vivo and wherein the differentially expressed marker is releasable from the platelets after activation in vitro.

7. The method according to any of claims 1 to 6, wherein the marker of platelet function is CD40L, sCD40L, P-selectin, soluble P-selectin, GP-IV, soluble GP-IV, GP-V, soluble GP-V, GP-VI, soluble GP-VI, CD63, platelet factor-4 (PF-4), β-Thromboglobulin (β-TG), or any variant thereof.

8. The method according to any of claims 1 to 7, wherein the method is used for diagnosing the degree of in vivo platelet stimulation.

9. The method according to any of claims 1 to 7, wherein the method is used for diagnosing platelet hypo- or hyperfunction.

10. The method according to claim 9, comprising the step of comparing the measured level of the marker of platelet function with a reference level indicative of platelet hypo- or hyperfunction.

11. The method according to any of claims 1 to 7, wherein the method is used for diagnosing a disorder diagnosable by measuring the level of sCD40L present on the surface of platelets.

12. The method according to claim 11, wherein the disorder is chosen from the group consisting of cardiovascular disorders, heparin-induced thrombocytopenia (HIT), thrombocytopenic thrombotic purpura (TTP), HELLP syndrome, and thrombotic microangiopathy.

13. The method according to any of claims 1 to 12, wherein step b) also comprises keeping the sample under conditions capable of inhibiting fibrinolysis.

14. The method according to claim 13, wherein the sample is kept under the conditions capable of inhibiting fibrinolysis by contacting the sample with an inhibitor of fibrinolysis.

15. A blood sampling device comprising
a) an anticoagulant,
b) a platelet activator and/or a protease,
c) optionally an inhibitor of platelet activation and/or a proteolysis inhibitor, and
d) optionally an inhibitor of fibrinolysis.

16. The device according to claim 15, wherein the device comprises a container with an intake portion for the intake of a blood sample and a reservoir portion for receiving the sample.

17. The device according to any of claims 15 to 16, wherein the amount of the platelet activator contained in the device for activating the platelets is sufficient to result in shedding of at least 30% of sCD40L from the total amount of CD40L present of the platelet surface within less than 10 minutes in an amount of blood for which said reservoir portion is designed.

18. The device according to any of claims 15 to 17, wherein the amount of the platelet activator contained in the device for activating the platelets is sufficient to have at least the platelet activating ability of 0.1 µg/ml of collagen or 0.1 µM of ADP in a blood sample.

19. The device according to any of claims 15 to 18, wherein the device comprises the inhibitor of platelet function and/or the proteolysis inhibitor in a mode that enables a release of said inhibitor(s) into a blood-platelet containing sample after the addition of the blood platelet-containing sample and after bringing the marker of platelet function or variant thereof into solution.

20. A method of manufacturing a device according to any of claims 15 to 19, comprising the steps of
a) providing container suitable for use in a device for collecting a blood sample from a subject, said container preferably having a reservoir portion for receiving the sample,
b) adding a platelet activator to the container, preferably to the reservoir portion,
c) optionally adding an anticoagulant to the container, preferably to the reservoir portion of the container,
d) assembling the device.

21. An assay device comprising a device according to any of claims 16 to 19 and means for measuring the level of a marker of platelet function.

22. A kit for carrying out the method according to any of claims 1 to 14, comprising
a) a blood sampling device,
b) a platelet activator and/or a protease,
c) optionally, an anticoagulant.
d) optionally an inhibitor of platelet activation and/or a proteolysis inhibitor, and
e) optionally an inhibitor of fibrinolysis.

23. A composition comprising
a) at least one anticoagulant, and
b) at least one platelet activator.
